# EUROPEAN PATENT APPLICATION

(11) **EP 4 685 212 A1**
(43) Date of publication of application: **28.01.2026**
(21) Application number: 24315356.6
(22) Date of filing: 25.07.2024
(51) Int. Cl.: C10G 11/18, C07C 6/04, C10G 35/09, C10G 51/06, C10G 59/02, C10G 67/08

(54) **INTEGRATED HSFCC PROCESSES FOR PRODUCING CHEMICAL INTERMEDIATES FROM CRUDE OIL**

(71) Applicant: Saudi Arabian Oil Company, Dhahran, 31311 (SA); Axens, 92500 Rueil Malmaison (FR); Technip Energies, Houston, TX 77079 (US); IFP Energies nouvelles, 69360 Solaize (FR)
(72) Inventor: Albaity, Abdullah S., 31311 Dhahran (SA); Ballesteros, Alberto Lozano, 31311 Dhahran (SA); Ghadeer, Abdulelah S., 31311 Dhahran (SA); Killingworth, Marcus, 31311 Dhahran (SA); Al Amoudi, Omar M., 31311 Dhahran (SA); Lambert, Marie-Amelie, 92500 Rueil-Malmaison (FR); Golczynski, Scott, Houston, TX 77079 (US); Verstraete, Jan, 69360 Solaize (FR)
(74) Representative: Yes My Patent

(57) **Abstract**

Integrated processes for converting a crude oil to light olefins and light aromatics includes processing a crude oil in a high severity fluidized catalytic cracking (HSFCC) system that includes two reactors, and separating a HSFCC effluent to produce a light products effluent, a mixed C₄ effluent, a naphtha effluent, and a heavy effluent. The process includes separating the light products effluent in an olefin separation system into a light olefin effluent and a light saturated hydrocarbon effluent and subjecting the light saturated hydrocarbon effluent to steam cracking. The method further includes passing the mixed C₄ effluent to a C₄ processing system to produce light olefins. The process includes reforming the naphtha effluent to produce a reformate and recovering mixed xylenes from the reformate. The process includes passing a portion of the heavy effluent to a heavy oil processing system to produce light cycle oil and heavy cycle oil.

## Description

### TECHNICAL FIELD

Embodiments of the present disclosure generally relate to processes for converting hydrocarbon feeds to chemical intermediates, in particular, processes integrating high severity fluidized catalytic cracking (HSFCC) technology and refinery process for upgrading crude oil to produce light olefins and light aromatic compounds.

### BACKGROUND

Light olefins, such as ethylene, propene, butene, butadiene, and light aromatic compounds such as benzene, toluene and xylenes are basic intermediates for the petrochemical industry. These compounds can be produced through refinery fluidized catalytic cracking (FCC) processes. The worldwide increasing demand for light olefins and light aromatics remains a major challenge for many integrated refineries. In particular, the production of some valuable light olefins such as ethylene, propene, and butene has attracted increased attention as purified olefin streams are considered the building blocks for polymer synthesis. The production of light olefins and light aromatic compounds depends on several process variables, such as the feed type, operating conditions, and the type of catalyst.

### SUMMARY

To satisfy the increasing need for light olefins, there is a need for improved refinery processes to produce light olefins and light aromatic compounds. Embodiments of the present disclosure are directed to integrated processes for upgrading a crude oils to produce one or more petrochemical products, such as light olefins and light aromatics. In particular, the processes of the present disclosure include a dual-downer HSFCC unit integrated with a petrochemical complex. The processes of the present disclosure may achieve at least 60 wt.% conversion of crude oil to light olefins based on the total flow rate of the crude oil feed.

According to embodiments, an integrated process for upgrading a crude oil includes processing a crude oil in a high severity fluidized catalytic cracking (HSFCC) system to produce an HSFCC effluent. The HSFCC system comprises a feed separator, a first FCC unit, and a second FCC unit in parallel with the first FCC unit. The process further includes separating the HSFCC effluent in a separation system to produce, a light products effluent, a mixed C4 effluent, a naphtha effluent, and a heavy effluent. The light products effluent comprises hydrocarbon constituents of the HSFCC effluent having a boiling point temperature less than or equal to -20 °C. The mixed C4 effluent comprises constituents of the HSFCC effluent having 4 carbon atoms. The naphtha effluent comprises constituents of the HSFCC effluent having boiling point temperatures of from 25 °C to 220 °C. The heavy effluent comprises constituents of the HSFCC effluent having boiling point temperatures greater than 220 °C. The process further includes passing the light products effluent to an olefin separation system that separates the light products effluent to produce a C2-C3 olefin effluent and a light saturated hydrocarbon effluent. The C2-C3 olefin effluent comprises ethylene, propylene, or both. The process further includes subjecting the light saturated hydrocarbon effluent to steam cracking in a pyrolysis cracking system to produce a cracking reaction effluent comprising ethylene, propylene, or both. The process further includes passing at least a portion of the mixed C4 effluent to a C4 processing system that processes the mixed C4 effluent to produce one or more of a saturated C4 stream and a C4 olefin stream. The process further includes reforming the naphtha effluent in a naphtha reforming system to produce a reformate comprising a greater concentration of light aromatic compounds compared to the naphtha effluent. The light aromatic compounds comprise benzene, toluene, xylenes, ethylbenzene, or combinations thereof. The process further includes recovering benzene, toluene, xylenes, ethylbenzene, or combinations thereof from the reformate. The process further includes passing at least a portion of the heavy effluent to heavy oil processing system that processes the heavy effluent to produce a light cycle oil (LCO) and a heavy cycle oil (HCO).

### BRIEF DESCRIPTION OF THE DRAWINGS

The following detailed description of specific aspects of the present disclosure can be best understood when read in conjunction with the following drawings, in which like structure is indicated with like reference numerals and in which:
FIG. 1 schematically depicts a generalized flow diagram of an embodiment of a process integrating high severity fluidized catalytic cracking (HSFCC) with refinery processes for upgrading crude oil to greater value chemical intermediates, according to one or more embodiments described in this disclosure;
FIG. 2 schematically depicts a generalized flow diagram of an embodiment of a HSFCC system of the process of FIG. 1, according to one or more embodiments described in this disclosure;
FIG. 3 schematically depicts a generalized flow diagram of another embodiment of a HSFCC system for the process of FIG. 1, according to one or more embodiments described in this disclosure;
FIG. 4 schematically depicts a generalized flow diagram of a pyrolysis cracking system of the process of FIG. 1, according to embodiments shown and described in the present disclosure;
FIG. 5 schematically depicts a generalized flow diagram of one embodiment of a C₄ processing system of the process of FIG. 1, according to embodiments shown and described in the present disclosure;
FIG. 6 schematically depicts a generalized flow diagram of another embodiment of a C₄ processing system of the process of FIG. 1, according to embodiments shown and described in the present disclosure;
FIG. 7 schematically depicts a generalized flow diagram of a naphtha reforming system of the process of FIG. 1, according to embodiments shown and described in the present disclosure;
FIG. 8 schematically depicts a generalized flow diagram of an aromatic recovery complex of the process of FIG. 1, according to embodiments shown and described in the present disclosure; and
FIG. 9 schematically depicts a generalized flow diagram of a heavy oil processing system of the process of FIG. 1, according to embodiments shown and described in the present disclosure.

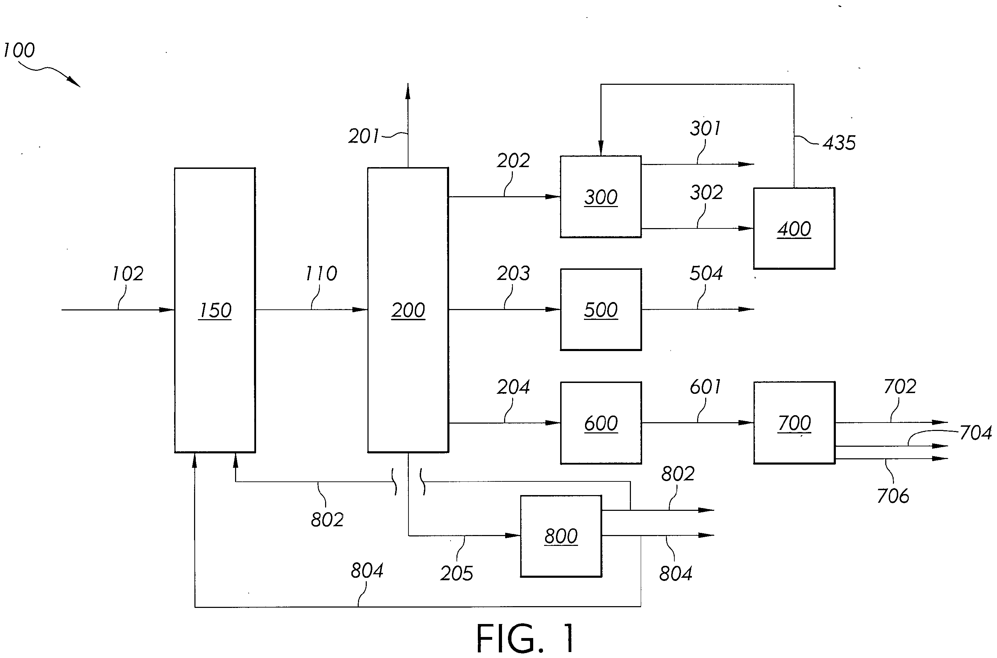

For purposes of describing the simplified schematic illustrations and descriptions in FIGS. 1-9, the numerous valves, temperature sensors, flow meters, pressure regulators, electronic controllers, pumps, heat exchangers, and the like that may be employed and well known to those of ordinary skill in the art of certain chemical processing operations may not be depicted. Further, accompanying components that are often included in typical chemical processing operations, such as valves, pipes, pumps, agitators, heat exchangers, condensers, boilers, instrumentation, internal vessel structures, or other subsystems may not be depicted. Though not depicted, it should be understood that these components are within the spirit and scope of the present embodiments disclosed. However, operational components, such as those described in the present disclosure, may be added to the embodiments described in this disclosure.

It should further be noted that arrows in the drawings refer to process streams. However, the arrows may equivalently refer to transfer lines which may serve to transfer process streams between two or more system components. Additionally, arrows that connect to system components define inlets or outlets in each given system component. The arrow direction corresponds generally with the major direction of movement of the materials of the stream contained within the physical transfer line signified by the arrow. Furthermore, arrows which do not connect two or more system components signify a product stream which exits the depicted system or a system inlet stream which enters the depicted system. Product streams may be further processed in accompanying chemical processing systems or may be commercialized as end products. System inlet streams may be streams transferred from accompanying chemical processing systems or may be non-processed feedstock streams. Some arrows may represent recycle streams, which are effluent streams of system components that are recycled back into the system. However, it should be understood that any represented recycle stream, in embodiments, may be replaced by a system inlet stream of the same material, and that a portion of a recycle stream may exit the system as a system product.

Additionally, arrows in the drawings may schematically depict process steps of transporting a stream from one system component to another system component. For example, an arrow from one system component pointing to another system component may represent "passing" a system component effluent to another system component, which may include the contents of a process stream "exiting" or being "removed" from one system component and "introducing" the contents of that product stream to another system component.

It should be understood that two or more process streams are "mixed" or "combined" when two or more lines intersect in the schematic flow diagrams of FIGS. 1-9. Mixing or combining may also include mixing by directly introducing both streams into a like reactor, separation device, or other system component. For example, it should be understood that when two streams are depicted as being combined directly prior to entering a separation unit or reactor, that in some embodiments the streams could equivalently be introduced into the separation unit or reactor and be mixed in the reactor. Dashed boxes drawn around multiple units are included in the drawings to indicate groupings of unit operations into subsystems.

Reference will now be made in greater detail to various aspects of the present disclosure, some aspects of which are illustrated in the accompanying drawings.

### DETAILED DESCRIPTION

The present disclosure is directed to processes for converting hydrocarbons in a crude oil feed to produce light olefins, light aromatics, or both. Referring to FIG. 1, one embodiment of a system 100 for converting a hydrocarbon feed stream 102 to greater value chemical intermediates, such as but not limited to light olefins, light aromatics, or both is schematically depicted. The system 100 may include an HSFCC system 150, a separation unit 200 downstream of the HSFCC system 150, an olefin separation system 300, a pyrolysis cracking system 400, a C4 processing system 500, a naphtha reforming system 600, an aromatics recovery complex 700, a heavy oil processing system 800, and any combinations of these systems. The HSFCC system 150 may be operable to crack a hydrocarbon feed stream 102, such as a crude oil stream, to produce an HSFCC effluent 110. The HSFCC effluent 110 may be separated into fractions by the separation unit 200 downstream of the HSFCC system 150. The HSFCC effluent 110 may be separated into at least a light products effluent 202, a mixed C₄ effluent 203, a naphtha effluent 204, and a heavy effluent 205. The light products effluent 202 may be passed to the olefin separation system 300 that produces a C₂-C₃ olefin effluent 301 and a light saturated hydrocarbon effluent 302. The light saturated hydrocarbon effluent 302 may be passed to the pyrolysis cracking system 400 to produce a cracking reaction effluent 435 comprising light olefins such as ethylene, propylene, or both. The cracking reaction effluent 435 may be passed back to the olefin separation system 300. The mixed C₄ effluent 203 may be passed to the C₄ processing system 500 to produce at least C₄ olefins 504. The naphtha effluent 204 may be passed to the naphtha reforming system 600 to produce a reformate 601. The reformate 601 may be passed downstream to the aromatic recovery complex 700 to recover light aromatics, such as but not limited to least mixed xylenes 701, from the reformate 601. The heavy effluent 205 may be passed to the heavy oil processing system 800 that processes the heavy effluent 205 to produce at least a light cycle oil 804.

The system 100 may be used in an integrated process for upgrading a crude oil, such as the hydrocarbon feed stream 102, into greater value chemical intermediates, such as but not limited to light olefins, light aromatics, or both. The process may include processing a crude oil 102 in an HSFCC system 150 to produce an HSFCC effluent 110, wherein the HSFCC system 150 comprises a feed separator 104, a first FCC unit 120, and a second FCC unit 150 in parallel with the first FCC unit. The process may further include separating the HSFCC effluent 110 in a separation system 200 to produce, a light products effluent 202, a mixed C₄ effluent 203, a naphtha effluent 204, and a heavy effluent 205. The light products effluent 202 comprises hydrocarbon constituents of the HSFCC effluent 110 having a boiling point temperature less than or equal to - 20 °C. The mixed C₄ effluent 203 comprises constituents of the HSFCC effluent 110 having boiling point temperatures of from -20 °C to 25 °C. The naphtha effluent 204 comprises constituents of the HSFCC effluent 110 having boiling point temperatures of from 25 °C to 220 °C. The heavy effluent 205 comprises constituents of the HSFCC effluent 110 having boiling point temperatures greater than 220 °C. The process may further include passing the light products effluent 202 to an olefin separation system 300 that separates the light products effluent to produce a C₂-C₃ olefin effluent 301 and a light saturated hydrocarbon effluent 302, where the C₂-C₃ olefin effluent comprises ethylene, propylene, or both. The process may further include subjecting the light saturated hydrocarbon effluent 302 to steam cracking in a pyrolysis cracking system 400 to produce a cracking reaction effluent 435 comprising ethylene, propylene, or both. The process may further include passing at least a portion of the mixed C₄ effluent 203 to a C₄ processing system 500 that processes the mixed C₄ effluent 203 to produce at least a C₄ olefin stream 504. The C₄ olefin stream 504 may comprise unsaturated C₄ hydrocarbons. The process may further include reforming the naphtha effluent 204 in a naphtha reforming system 600 to produce a reformate 601 comprising a greater concentration of light aromatic compounds compared to the naphtha effluent 204, where the light aromatic compounds comprise benzene, toluene, xylenes, ethylbenzene, or combinations thereof. The process may further include recovering benzene, toluene, xylenes, ethylbenzene, or combinations thereof from the reformate 601. The process may further include passing at least a portion of the heavy effluent 205 to heavy oil processing system 800 that processes the heavy effluent to produce a light cycle oil (LCO) 802 and a heavy cycle oil (HCO) 804.

Embodiments of the present disclosure are directed to processes for converting hydrocarbons to produce light olefins. Various embodiments are discussed herein. However, it should be understood that the forgoing detailed description section describes one or more specific embodiments and should not be viewed as limiting the scope of the appended claims.

The indefinite articles "a" and "an" are employed to describe elements and components of the present disclosure. The use of these articles means that one or at least one of these elements or components is present. Although these articles are conventionally employed to signify that the modified noun is a singular noun, as used herein the articles "a" and "an" also include the plural, unless otherwise stated in specific instances. Similarly, the definite article "the", as used in the present disclosure, also signifies that the modified noun may be singular or plural, unless otherwise stated in specific instances.

As used in this disclosure, passing a stream or effluent from one unit "directly" to another unit refers to passing the stream or effluent from the first unit to the second unit without passing the stream or effluent through an intervening reaction system or separation system that substantially changes the composition of the stream or effluent, such as through chemical reaction or through preferential separation of one or more constituents from the stream or effluent. Heat transfer devices, such as heat exchangers, preheaters, coolers, condensers, or other heat transfer equipment, and pressure devices, such as pumps, pressure regulators, compressors, or other pressure devices, are not considered to be intervening systems that change the composition of a stream or effluent, unless otherwise specifically stated in the present disclosure. Combining two streams or effluents together upstream of a process unit also is not considered to comprise an intervening system that changes the composition of one or both of the streams or effluents being combined. Simply dividing a stream into two streams having the same composition is also not considered to comprise an intervening system that changes the composition of the stream.

As used in the present disclosure, the term "fluid" refers to a flowable composition that includes gases, liquids, or a combination of these.

As used in this disclosure, a "reactor" refers to a vessel in which one or more chemical reactions may occur between one or more reactants optionally in the presence of one or more catalysts. For example, a reactor may include a tank or tubular reactor configured to operate as a batch reactor, a continuous stirred-tank reactor (CSTR), packed bed reactor, fluidized bed reactor, a plug flow reactor, or other type of reactor. Example reactors include packed bed reactors, such as fixed bed reactors, and fluidized bed reactors. One or more "reaction zones" may be disposed in a reactor. As used in this disclosure, a "reaction zone" refers to an area where a particular reaction takes place in a reactor.

As used in this disclosure, a "separation unit" refers to any separation device that at least partially separates one or more chemicals that are mixed in a process stream from one another. For example, a separation unit may selectively separate differing chemical species from one another, forming one or more chemical fractions. Examples of separation units include, without limitation, distillation columns, flash drums, knock-out drums, knock-out pots, centrifuges, cyclones, filtration devices, traps, scrubbers, expansion devices, membranes, solvent extraction devices, and the like. It should be understood that separation processes described in this disclosure may not completely separate all of one chemical constituent from all of another chemical constituent. It should be understood that the separation processes described in this disclosure "at least partially" separate different chemical components from one another, and that even if not explicitly stated, it should be understood that separation may include only partial separation. As used in this disclosure, one or more chemical constituents may be "separated" from a process stream to form a new process stream. Generally, a process stream may enter a separation unit and be divided, or separated, into two or more process streams of desired composition. Further, in some separation processes, a "lesser boiling point fraction" (sometimes referred to as a "light fraction") and a "greater boiling point fraction" (sometimes referred to as a "heavy fraction") may exit the separation unit, where, on average, the contents of the lesser boiling point fraction have a lesser boiling point than the greater boiling point fraction. Other streams may fall between the lesser boiling point fraction and the greater boiling point fraction, such as an "intermediate boiling point fraction."

As used in this disclosure, the term "high severity conditions" generally refers to FCC temperatures of 500 °C or greater, a weight ratio of catalyst to hydrocarbon (catalyst to oil ratio) of equal to or greater than 5:1, and a residence time of less than or equal to 3 seconds, all of which may be more severe than reaction conditions in conventional FCC units.

It should be understood that an "effluent" generally refers to a stream that exits a system component such as a separation unit, a reactor, or reaction zone, following a particular reaction or separation, and generally has a different composition (at least proportionally) than the stream that entered the separation unit, reactor, or reaction zone.

As used in this disclosure, a "catalyst" refers to any substance which increases the rate of a specific chemical reaction. Catalysts described in this disclosure may be utilized to promote various reactions, such as, but not limited to, cracking (including but not limited to fluidized catalytic cracking, aromatic cracking, steam cracking, etc.), hydrotreating (including but not limited to demetalization, desulfurization, and denitrogenation), hydrogenation, reforming (including but not limited to dehydrocyclization, dehydrogenation, isomerization), metathesis, or other chemical reactions.

As used in this disclosure, "cracking" generally refers to a chemical reaction where a molecule having carbon to carbon bonds is broken into more than one molecule by the breaking of one or more of the carbon to carbon bonds, or is converted from a compound which includes a cyclic moiety, such as a cycloalkane, cycloalkane, naphthalene, an aromatic, or the like, to a compound which does not include a cyclic moiety or contains fewer cyclic moieties than prior to cracking.

As used in this disclosure, the term "spent catalyst" refers to catalyst that has been contacted with reactants but has not been regenerated to restore at least a portion of the catalytic activity. For instance, a "spent cracking catalyst" refers to a cracking catalyst that has been introduced to and passed through a cracking reaction zone to crack a hydrocarbon material, such as the greater boiling point fraction or the lesser boiling point fraction for example, but has not been regenerated in the regenerator following introduction to the cracking reaction zone. The "spent catalyst" may have coke deposited on the catalyst and may include partially coked catalyst as well as fully coked catalysts. The amount of coke deposited on the "spent catalyst" may be greater than the amount of coke remaining on the regenerated catalyst following regeneration. The "spent catalyst" may also have a reduced temperature compared to the regenerated catalyst following regeneration.

As used in this disclosure, the term "regenerated catalyst" refers to catalyst that has been contacted with reactants in a reaction zone at reaction conditions and then regenerated in a regenerator or through a regeneration process to increase the catalytic activity, the temperature, or both of the regenerated catalyst. The "regenerated catalyst" may have less coke, a greater temperature, or both compared to spent catalyst and may have greater catalytic activity compared to spent catalyst. The "regenerated catalyst" may have more coke and lesser catalytic activity compared to fresh catalyst that has not passed through a cracking reaction zone and regenerator.

As used in this disclosure, the term "fresh catalyst" refers to catalyst that has not been previously contacted with reactants at reaction conditions in a reaction zone.

As used in this disclosure, the term "aromatic compounds" refers to compounds having one or more aromatic ring structures. The term "light aromatic compounds" refers to compounds having an aromatic ring, with or without substitution, and from six to eight carbon atoms. The term "BTEX" refers to any combination of one or a plurality of benzene, toluene, ethylbenzene,para-xylene, meta-xylene, and ortho-xylene.

As used in this disclosure, the terms "xylenes" and "mixed xylenes" when used without a designation of the isomer, such as the prefix *para, meta,* or *ortho,* refers to one or more of meta-xylene, ortho-xylene, para-xylene, and mixtures of these xylene isomers.

As used in this disclosure, the terms "butenes" and "mixed butenes" refers to 1-butene, cis-2-butene, *trans*-2-butene, isobutene, and combinations of these. As used in this disclosure, the term "normal butenes" refers to 1-butene, cis-2-butene, *trans*-2-butene, and any combination thereof, but not including isobutene.

As used in this disclosure, the terms "C4," "C4 hydrocarbons," or "C4 compounds" refers to hydrocarbon compounds having 4 carbon atoms.

As used in this disclosure, the terms "upstream" and "downstream" refer to the relative positioning of unit operations with respect to the direction of flow of the process streams through the system. A first unit operation of a system is considered "upstream" of a second unit operation if process streams flowing through the system encounter the first unit operation before encountering the second unit operation. Likewise, a second unit operation is considered "downstream" of the first unit operation if the process streams flowing through the system encounter the first unit operation before encountering the second unit operation.

As used in this disclosure, the terms "atmospheric boiling point temperature," "boiling point temperature," "boiling temperature," and "boiling point," all refer to the boiling point temperature of a compound at atmospheric pressure, unless otherwise specifically stated.

As used in this disclosure, the term "initial boiling point" or "IBP" of a composition refers to the temperature at which the constituents of the composition with the least boiling point temperatures begin to transition from the liquid phase to the vapor phase. As used in this disclosure, the term "end boiling point" or "EBP" of a composition refers to the temperature at which the greatest boiling temperature constituents of the composition transition from the liquid phase to the vapor phase. A hydrocarbon mixture may be characterized by a distillation profile expressed as boiling point temperatures at which a specific weight percentage of the composition has transitioned from the liquid phase to the vapor phase.

It should be understood that streams may be named for the components of the stream, and the component for which the stream is named may be the major component of the stream (such as comprising from 50 weight percent (wt. %), from 70 wt. %, from 90 wt. %, from 95 wt. %, from 99 wt. %, from 99.5 wt. %, or even from 99.9 wt. % of the contents of the stream to 150 wt. % of the contents of the stream). It should also be understood that components of a stream are disclosed as passing from one system component to another when a stream comprising that component is disclosed as passing from that system component to another. For example, a disclosed "greater boiling point fraction" passing from a first system component to a second system component should be understood to equivalently disclose the "greater boiling point fraction" passing from a first system component to a second system component.

### Hydrocarbon Feed Stream

Referring again to FIG. 1, the hydrocarbon feed stream 102 may generally comprise a hydrocarbon material. In embodiments, the hydrocarbon material of the hydrocarbon feed stream may be crude oil, such as but not limited to an Arab extra light crude oil (AXL). As used in this disclosure, the term "crude oil" is to be understood to mean a mixture of petroleum liquids and gases, including one or more impurities such as sulfur-containing compounds, nitrogen-containing compounds and metal compounds, as distinguished from fractions of crude oil. In certain embodiments the crude oil feedstock may be a minimally treated light crude oil to provide a crude oil feedstock having total metals (Ni+V) content of less than 5 parts per million by weight (ppmw) and Conradson carbon residue of less than 5 wt %.

While the present description and examples may specify crude oil as the hydrocarbon material of the hydrocarbon feed stream 102, it should be-understood that the hydrocarbon feed conversion systems 150 described with respect to the embodiments of FIGS. 1-9, respectively, may be applicable for the conversion of a wide variety of hydrocarbon materials, which may be present in the hydrocarbon feed stream 102, including, but not limited to, crude oil, vacuum residue, tar sands, bitumen, atmospheric residue, vacuum gas oils, demetalized oils, naphtha streams, other hydrocarbon streams, or combinations of these materials. The hydrocarbon feed stream 102 may include one or more non-hydrocarbon constituents, such as one or more heavy metals, sulphur compounds, nitrogen compounds, inorganic components, or other non-hydrocarbon compounds. If the hydrocarbon feed stream 102 is crude oil, it may have an American Petroleum Institute (API) gravity of from 22 degrees to 50 degrees. For example, the hydrocarbon feed stream 102 utilized may be an Arab light crude oil or an Arab extra light crude oil. Example properties for one particular exemplary grade of Arab light crude oil are provided subsequently in Table 1. It should be understood that, as used in this disclosure, a "hydrocarbon feed" may refer to a raw hydrocarbon material which has not been previously treated, separated, or otherwise refined (such as crude oil) or may refer to a hydrocarbon material which has undergone some degree of processing, such as treatment, separation, reaction, purifying, or other operation, prior to being introduced to the HSFCC system 150 in the hydrocarbon feed stream 102.

The hydrocarbon feed stream 102 may include one or more heavy oils, such as but not limited to crude oil, bitumen, oil sand, shale oil, coal liquids, vacuum residue, tar sands, other heavy oil streams, or combinations of these. It should be understood that, as used in this disclosure, a "heavy oil" refers to a raw hydrocarbon, such as whole crude oil, which has not been previously processed through distillation, or may refer to a hydrocarbon oil, which has undergone some degree of processing prior to being introduced to the process as the hydrocarbon feed 102. The hydrocarbon feed stream 102 may have a density of greater than or equal to 0.80 grams per milliliter. The hydrocarbon feed stream 102 may have an end boiling point (EBP) of greater than 565 °C. The hydrocarbon feed stream 102 may have a concentration of nitrogen of less than or equal to 3000 parts per million by weight (ppmw).

In embodiments, the hydrocarbon feed stream 102 may be a crude oil, such as whole crude oil, or synthetic crude oil. The crude oil may have an American Petroleum Institute (API) gravity of from 22 degrees to 50 degrees, such as from 22 degrees to 40 degrees, from 25 degrees to 50 degrees, or from 25 degrees to 40 degrees. For example, the hydrocarbon feed stream 102 may include an extra light crude oil, a light crude oil, a medium crude oil, a heavy crude oil, or combinations of these. In embodiments, the hydrocarbon feed stream 102 can be a light crude oil, such as but not limited to an Arab light export crude oil. Example properties for an exemplary grade of Arab light (AL) crude oil are provided in Table 1.

**Table 1 - Example of AL Export Feedstock**

| **Analysis** | **Units** | **Value** | **Test Method** |
|---|---|---|---|
| American Petroleum Institute (API) gravity | degree | 33.13 | ASTM D287 |
| Density | grams per milliliter (g/mL) | 0.8595 | ASTM D287 |
| Carbon Content | weight percent (wt.%) | 85.29 | ASTM D5291 |
| Hydrogen Content | wt.% | 12.68 | ASTM D5292 |
| Sulfur Content | wt.% | 1.94 | ASTM D5453 |
| Nitrogen Content | parts per million by weight (ppmw) | 849 | ASTM D4629 |
| Asphaltenes | wt.% | 1.2 | ASTM D6560 |
| Micro Carbon Residue (MCR) | wt. % | 3.4 | ASTM D4530 |
| Vanadium (V) Content | ppmw | 15 | IP 501 |
| Nickel (Ni) Content | ppmw | 12 | IP 501 |
| Arsenic (As) Content | ppmw | 0.04 | IP 501 |

| Boiling Point Dis tribution | | | |
|---|---|---|---|
| Initial Boiling Point (IBP) | Degrees Celsius (°C) | 33 | ASTM D7169 |
| 5% Boiling Point (BP) | °C | 92 | ASTM D7169 |
| 10% BP | °C | 133 | ASTM D7169 |
| 20% BP | °C | 192 | ASTM D7169 |
| 30% BP | °C | 251 | ASTM D7169 |
| 40% BP | °C | 310 | ASTM D7169 |
| 50% BP | °C | 369 | ASTM D7169 |
| 60% BP | °C | 432 | ASTM D7169 |
| 70% BP | °C | 503 | ASTM D7169 |
| 80% BP | °C | 592 | ASTM D7169 |
| 90% BP | °C | >720 | ASTM D7169 |
| 95% BP | °C | >720 | ASTM D7169 |
| End Boiling Point (EBP) | °C | >720 | ASTM D7169 |
| BP range C5-180 °C | wt.% | 18.0 | ASTM D7169 |
| BP range 180 °C - 350 °C | wt.% | 28.8 | ASTM D7169 |
| BP range 350 °C - 540 °C | wt.% | 27.4 | ASTM D7169 |
| BP range >540 °C | wt.% | 25.8 | ASTM D7169 |
| Weight percentages in Table 1 are based on the total weight of the crude oil. | | | |

In embodiments, the hydrocarbon feed stream 102 may be an Arab extra light (AXL) crude oil. An example boiling point distribution for an exemplary grade of an AXL crude oil is provided in Table 2.

**Table 2: Example of AXL feedstock**

| **Property** | **Units** | **Value** | **Test Method** |
|---|---|---|---|
| 0.1% Boiling Point (BP) | °C | 21 | ASTM D7169 |
| 5% BP | °C | 65 | ASTM D7169 |
| 10% BP | °C | 96 | ASTM D7169 |
| 15% BP | °C | 117 | ASTM D7169 |
| 20% BP | °C | 141 | ASTM D7169 |
| 25% BP | °C | 159 | ASTM D7169 |
| 30% BP | °C | 175 | ASTM D7169 |
| 35% BP | °C | 196 | ASTM D7169 |
| 40% BP | °C | 216 | ASTM D7169 |
| 45% BP | °C | 239 | ASTM D7169 |
| 50% BP | °C | 263 | ASTM D7169 |
| 55% BP | °C | 285 | ASTM D7169 |
| 60% BP | °C | 308 | ASTM D7169 |
| 65% BP | °C | 331 | ASTM D7169 |
| 70% BP | °C | 357 | ASTM D7169 |
| 75% BP | °C | 384 | ASTM D7169 |
| 80% BP | °C | 415 | ASTM D7169 |
| 85% BP | °C | 447 | ASTM D7169 |
| 90% BP | °C | 486 | ASTM D7169 |
| 95% BP | °C | 537 | ASTM D7169 |
| End Boiling Point (EBP) | °C | 618 | ASTM D7169 |

When the hydrocarbon feed stream 102 comprises a crude oil, the crude oil may be a whole crude or may be a crude oil that has undergone at least some processing, such as desalting, solids separation, scrubbing, or other process that does not change the composition of the hydrocarbons of the crude oil. For example, the hydrocarbon feed stream 102 may be a de-salted crude oil that has been subjected to a de-salting process. In embodiments, the hydrocarbon feed stream 102 may include a crude oil that has not undergone pretreatment, separation (such as distillation), or other operation or process that changes the hydrocarbon composition of the crude oil prior to introducing the crude oil to the system 100.

In embodiments, the hydrocarbon feed stream 102 can be a crude oil having a boiling point profile as described by the 5 wt. % boiling temperature, the 25 wt. % boiling temperature, the 50 wt. % boiling temperature, the 75 wt. % boiling temperature, and the 95 wt. % boiling temperature. These respective boiling temperatures correspond to the temperatures at which a given weight percentage of the hydrocarbon feed stream boils. In embodiments, the crude oil may have one or more of a 5 wt. % boiling temperature of less than or equal to 150 °C, a 25 wt. % boiling temperature of less than or equal to 225 °C or less than or equal to 200 °C, a 50 wt. % boiling temperature of less than or equal to 500 °C, less than or equal 450 °C, or less than or equal to 400 °C, a 75 wt. % boiling temperature of less than 600 °C, less than or equal to 550 °C, a 95 wt. % boiling temperature of greater than or equal to 550 °C or greater than or equal to 600 °C, or combinations of these. In embodiments, the crude oil may have one or more of a 5 wt. % boiling temperature of from 0 °C to 100 °C, a 25 wt. % boiling temperature of from 150 °C to 250 °C, a 50 wt. % boiling temperature of from 250 °C to 400 °C, a 75 wt. % boiling temperature of from 350 °C to 600 °C and an end boiling point temperature of from 500 °C to 1000 °C, such as from 500 °C to 800 °C.

### HSFCC System

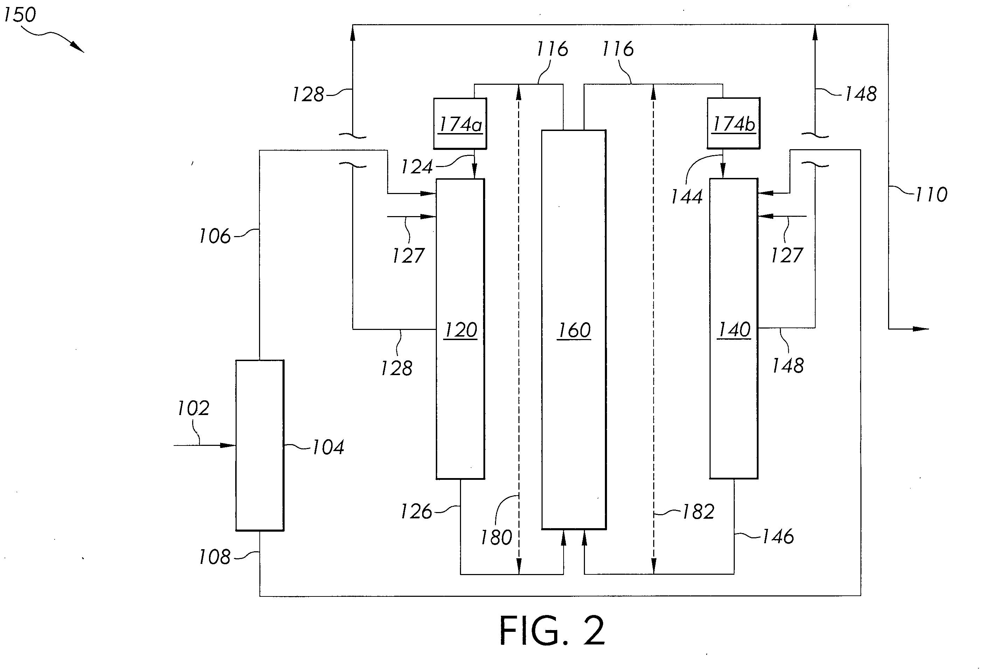

In general terms, the HSFCC system 150 includes two FCC units, in each of which a portion of the hydrocarbon feed stream 102 contacts heated fluidized catalytic particles in a cracking reaction zone maintained at high severity temperatures and pressures. Referring now to FIG. 2, one embodiment of the high severity fluid catalytic cracking (HSFCC) system 150 is schematically depicted. The HSFCC system 150 may receive the hydrocarbon feed stream 102, such as a crude oil stream, and may directly process the hydrocarbon feed stream 102 to produce an HSFCC effluent 110. The HSFCC system 150 may include a feed separator 104, a first FCC unit 120, a second FCC unit 140, and a regenerator 160.

The hydrocarbon feed stream 102 may be introduced to the feed separator 104, which may separate the hydrocarbon feed stream 102 into a greater boiling point fraction 106 and a lesser boiling point fraction 108. In embodiments, the feed separator 104 may be a vapor-liquid separator such as a flash drum (sometimes referred to as a breakpot, knock-out drum, knock-out pot, compressor suction drum, or compressor inlet drum). In embodiments that utilize a vapor-liquid separator as the feed separator 104, the lesser boiling point fraction 108 may exit the feed separator 104 as a vapor and the greater boiling point fraction 106 may exit the feed separator 104 as a liquid. The feed separator 104 may be operated at a temperature suitable to separate the hydrocarbon feed stream 102 into the greater boiling point fraction 106 and the lesser boiling point fraction 108. The temperature of the feed separator 104 may be from 180 degrees Celsius (°C) to 400 °C. For instance, the feed separator 104 may be operated at a cut-point temperature so that the constituents of the lesser boiling point fraction 108 may have boiling point temperatures of less than or equal to 400 °C, less than or equal to 350 °C, less than or equal to 300 °C, less than or equal to 250 °C, or less than or equal to 200 °C. The constituents of the greater boiling point fraction 106 may have boiling point temperatures greater than the boiling point of the contents of the lesser boiling point fraction 108. The constituents of the greater boiling point fraction 106 may have boiling point temperatures greater than the cut point temperature of the feed separator 104, such as boiling point temperatures of greater than 200 °C, greater than 250 °C, greater than 300 °C, greater than 350 °C, or even greater than 400 °C. In embodiments, the greater boiling point fraction 106 may have less than 10 wt.% Conradson Carbon based on the total weight of the greater boiling point fraction 106. In embodiments, the greater boiling point fraction 106 may have less than 10 parts per million by weight (PPMW) total metals based on the total weight of the greater boiling point fraction 106.

In embodiments, the feed separator 104 may be a flash column that may separate the hydrocarbon feed stream 102 into the greater boiling point fraction 106 and the lesser boiling point fraction stream 108. In embodiments, the flash column may be operated at a temperature of from 180 °C to 400 °C. In embodiments, the feed separator 104 may include at least one of a distillation device or a cyclonic vapor liquid separation device.

In embodiments, the hydrocarbon feed stream 102 may be introduced to the feed separator 104 and separated into the greater boiling point fraction 106 and the lesser boiling point fraction 108. Alternatively, in embodiments, the hydrocarbon feed stream 102 may comprise a plurality of refinery hydrocarbon streams, and the plurality of refinery hydrocarbon streams may be introduced directly to the first FCC unit 120, the second FCC unit 140, or both. For instance, one or more heavy refinery hydrocarbon streams, such as but not limited to vacuum residues, atmospheric residues, or vacuum gas oils, for example, may be introduced directly to the first FCC unit 120 as the greater boiling point fraction 106, and other light refinery hydrocarbon streams, such as but not limited to a naphtha stream, may be introduced directly to the second FCC unit 140 as the lesser boiling point fraction 108.

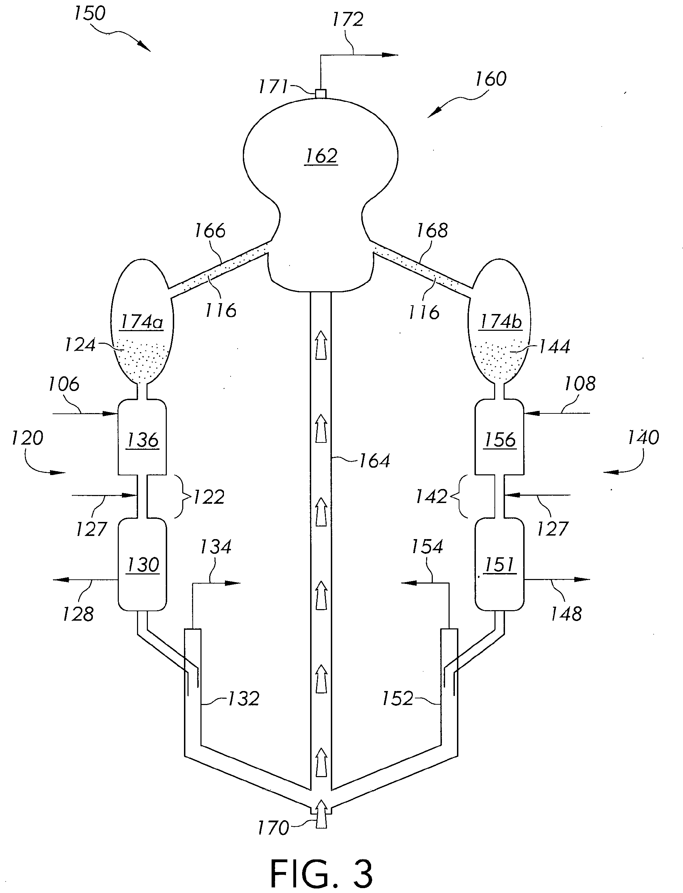

Referring now to FIGS. 2 and 3, the greater boiling point fraction 106 may be passed to a first FCC unit 120 that includes a first cracking reaction zone 122. The greater boiling point fraction 106 is combined or mixed with a first catalyst 124 and cracked to produce a mixture of a spent first catalyst 126 and a first cracking effluent 128. As used in this disclosure, the term "first catalyst" refers to at least one cracking catalyst that is introduced to the first cracking reaction zone, such as the catalyst passed from the first catalyst/feed mixing zone to the first cracking reaction zone. The first catalyst may include at least one of regenerated catalyst, spent first catalyst, spent second catalyst, fresh catalyst, or combinations of these. Steam 127 may be added to the first cracking reaction zone 122 to reduce the hydrocarbon partial pressure in the first cracking reaction zone 122. The spent first catalyst 126 is separated from the first cracking reaction product 128 and passed to a regeneration zone 162 of the regenerator 160.

Still referring to FIGS. 2 and 3, the lesser boiling point fraction 108 is passed to a second FCC unit 140 that includes a second cracking reaction zone 142. The lesser boiling point fraction 108 is mixed with a second catalyst 144 and cracked to produce a spent second catalyst 146 and a second cracking reaction product 148. As used in this disclosure, the term "second catalyst" refers to at least one cracking catalyst that is introduced to the second cracking reaction zone, such as the catalyst passed from the second catalyst/feed mixing zone to the second cracking reaction zone for example. The second catalyst may include at least one of regenerated catalyst, spent first catalyst, spent second catalyst, fresh catalyst, or combinations of these. Steam 127 may also be added to the second cracking reaction zone 142 to reduce the hydrocarbon partial pressure in the second cracking reaction zone 142. The spent second catalyst 146 is separated from the second cracking effluent 148 and passed to the regeneration zone 162 of the regenerator 160.

When the greater boiling point fraction 106 and the lesser boiling point fraction 108 contact the hot catalysts and are cracked to lighter products, carbonaceous deposits, commonly referred to as coke, may form on the catalysts. The coke deposits formed on the catalysts may reduce the catalytic activity of each of the catalysts or deactivate the catalysts. Deactivation of the catalyst may result in the catalyst becoming catalytically ineffective. The spent catalyst having coke deposits may be separated from the cracking reaction products, stripped of removable hydrocarbons, and passed to a regeneration process where the coke is burned from the catalyst in the presence of air to produce a regenerated catalyst that is catalytically effective. The term "catalytically effective" refers to the ability of the regenerated catalyst to increase the rate of cracking reactions. The term "catalytic activity" refers to the degree to which the regenerated catalyst increases the rate of the cracking reactions and may be related to a number of catalytically active sites available on the catalyst. For example, coke deposits on the catalyst may cover up or block catalytically active sites on the spent catalyst, thus, reducing the number of catalytically active sites available, which may reduce the catalytic activity of the catalyst. Following regeneration, the regenerated catalyst may have equal to or less than 1 wt.% coke based on the total weight of the regenerated catalyst. The combustion products may be removed from the regeneration process as a flue gas stream. The heated regenerated catalysts may then be recycled back to the cracking reaction zone of the FCC units.

The spent first catalyst 126 and the spent second catalyst 146 may be combined and regenerated in the regeneration zone 162 of the regenerator 160 to produce a regenerated catalyst 116. The regenerated catalyst 116 may have a catalytic activity that is at least greater than the catalytic activity of the spent first catalyst 126 and the spent second catalyst 146. The regenerated catalyst 116 may then be passed back to the first cracking reaction zone 122 and the second cracking reaction zone 142. The first cracking reaction zone 122 and the second cracking reaction zone 142 may be operated in parallel.

Referring to FIG. 2, in embodiments, the HSFCC system 150 may include at least one catalyst recycle, such as spent first catalyst recycle 180, spent second catalyst recycle 182, or both, for example. The catalyst recycles 180, 182 may be used to recycle the spent first catalyst 126, the spent second catalyst 146, or both to the first FCC unit 120, the second FCC unit 140, or both. The spent first catalyst 126, the spent second catalyst 146, or both may be combined with regenerated catalyst 116 to produce the first catalyst 124 to reduce the temperature, catalytic activity, or both of the first catalyst 124 relative to the regenerated catalyst 116. Similarly, the spent first catalyst 126, spent second catalyst 146, or both may be combined with the regenerated catalyst 116 to reduce the temperature, catalytic activity, or both of the second catalyst 144 relative to the regenerated catalyst 116.

The first cracking effluent 128 (FIG. 2, FIG. 3) and the second cracking effluent 148 (FIG. 2, FIG. 3) each may include a mixture of cracked hydrocarbon materials, such as but not limited to light gases, catalytic cracked naphtha, light cycle oil (LCO, 216-343 °C), heavy cycle oil (HCO, >343 °C), or combinations of these cracked hydrocarbon materials. In embodiments, the mixture of cracked hydrocarbon materials may include but are not limited to cracked gas oil, cracked gasoline, cracked naphtha, mixed butenes, butadiene, propene, ethylene, other olefins, ethane, methane, other petrochemical products, or combinations of these. The first cracking effluent 128 and the second cracking effluent 148 may be combined to produce an HSFCC effluent 110 (FIG. 2).

Referring again to FIG. 3, the first FCC unit 120 may include a first catalyst/feed mixing zone 136, the first cracking reaction zone 122, a first separation zone 130, and a first stripping zone 132. The greater boiling point fraction 106 may be introduced to the first catalyst/feed mixing zone 136, where the greater boiling point fraction 106 may be mixed with the first catalyst 124. During steady state operation of the HSFCC system 150, the first catalyst 124 may include at least the regenerated catalyst 116 that is passed to the first catalyst/feed mixing zone 136 from a first catalyst hopper 174a. In embodiments, the first catalyst 124 may be a mixture of spent first catalyst 126 and regenerated catalyst 116. Alternatively, the first catalyst 124 may be a mixture of spent second catalyst 146 and regenerated catalyst 116. The first catalyst hopper 174a may receive the regenerated catalyst 116 from the regenerator 160. At initial start-up of the HSFCC system 150, the first catalyst 124 may include fresh catalyst (not shown), which is catalyst that has not been circulated through the first FCC unit 120 or the second FCC unit 140 and the regenerator 160. Because the fresh catalyst has not been circulated through a cracking reaction zone, the fresh catalyst may have a catalytic activity that is greater than the regenerated catalyst 116. In embodiments, fresh catalyst may also be introduced to the first catalyst hopper 174a during operation of the HSFCC system 150 so that a portion of the first catalyst 124 introduced to the first catalyst/feed mixing zone 136 includes fresh catalyst. Fresh catalyst may be introduced to the first catalyst hopper 174a periodically during operation to replenish lost catalyst or compensate for spent catalyst that becomes deactivated, such as through heavy metal accumulation in the catalyst.

The mixture comprising the greater boiling point fraction 106 and the first catalyst 124 may be passed from the first catalyst/feed mixing zone 136 to the first cracking reaction zone 122. The mixture of the greater boiling point fraction 106 and first catalyst 124 may be introduced to a top portion of the first cracking reaction zone 122. The first cracking reaction zone 122 may be a downflow reactor or "downer" reactor in which the reactants flow from the first catalyst/feed mixing zone 136 vertically downward through the first cracking reaction zone 122 to the first separation zone 130. Steam 127 may be introduced to the top portion of the first cracking reaction zone 122 to reduce the hydrocarbon partial pressure of the mixture of the greater boiling point fraction 106 and the first catalyst 124. The greater boiling point fraction 106 may be reacted by contact with the first catalyst 124 in the first cracking reaction zone 122 to cause at least a portion of the greater boiling point fraction 106 to undergo at least a cracking reaction to form at least one cracking reaction product, which may include at least one of the petrochemical products previously described. The first catalyst 124 may have a temperature equal to or greater than the first cracking reaction zone 122 and may transfer heat to the greater boiling point fraction 106 to promote the endothermic cracking reaction.

It should be understood that the first cracking reaction zone 122 of the first FCC unit 120 depicted in FIG. 3 is a simplified schematic of one particular embodiment of the first cracking reaction zone 122 of an FCC unit, and other configurations of the first cracking reaction zone 122 may be suitable for incorporation into the HSFCC system 150. In embodiments, the first cracking reaction zone 122 may be an up-flow cracking reaction zone. Other cracking reaction zone configurations are contemplated. The first FCC unit 120 may be a HSFCC unit in which, in the first cracking reaction zone 122, the fluidized first catalyst 124 contacts the greater boiling point fraction 106 under high severity conditions. The temperature of the first cracking reaction zone 122 may be from 500 °C to 800 °C, from 500 °C to 700 °C, from 500 °C to 650 °C, from 500 °C to 600 °C, from 550 °C to 800 °C, from 550 °C to 700 °C, from 550 °C to 650 °C, from 550 °C to 600 °C, from 600 °C to 800 °C, from 600 °C to 700 °C, or from 600 °C to 650 °C. In embodiments, the temperature of the first cracking reaction zone 122 may be from 500 °C to 700 °C. In embodiments, the temperature of the first cracking reaction zone 122 may be from 550 °C to 630 °C.

A weight ratio of the first catalyst 124 to the greater boiling point fraction 106 in the first cracking reaction zone 122 (the catalyst to hydrocarbon ratio) may be from 5:1 to 40:1, from 5:1 to 35:1, from 5:1 to 30:1, from 5:1 to 25:1, from 5:1 to 15:1, from 5:1 to 10:1, from 10:1 to 40:1, from 10:1 to 35:1, from 10:1 to 30:1, from 10:1 to 25:1, from 10:1 to 15:1, from 15:1 to 40:1, from 15:1 to 35:1, from 15:1 to 30:1, from 15:1 to 25:1, from 25:1 to 40:1, from 25:1 to 35:1, from 25:1 to 30:1, or from 30:1 to 40:1. The residence time of the mixture of first catalyst 124 and the greater boiling point fraction 106 in the first cracking reaction zone 122 may be from 0.2 seconds (sec) to 3 sec, from 0.2 sec to 2.5 sec, from 0.2 sec to 2 sec, from 0.2 sec to 1.5 sec, from 0.4 sec to 3 sec, from 0.4 sec to 2.5 sec, or from 0.4 sec to 2 sec, from 0.4 sec to 1.5 sec, from 1.5 sec to 3 sec, from 1.5 sec to 2.5 sec, from 1.5 sec to 2 sec, or from 2 sec to 3 sec.

Following the cracking reaction in the first cracking reaction zone 122, the contents of the effluent from the first cracking reaction zone 122 may include the first catalyst 124 and the first cracking effluent 128, which may then be passed to the first separation zone 130. In the first separation zone 130, the first catalyst 124 may be separated from at least a portion of the first cracking effluent 128. In embodiments, the first separation zone 130 may include one or more gas-solid separators, such as one or more cyclones. The first catalyst 124 exiting from the first separation zone 130 may retain at least a residual portion of the first cracking effluent 128.

After the first separation zone 130, the first catalyst 124, which may include the residual portion of the first cracking reaction product 128 retained in the first catalyst 124, may be passed to a first stripping zone 132, where at least some of the residual portion of the first cracking effluent 128 may be stripped from the first catalyst 124 and recovered as a first stripped product stream 134. Steam may be introduced to the first stripping zone 132 to facilitate stripping the first cracking reaction product 128 from the first catalyst 124. The first stripped product stream 134 may include at least a portion of the steam introduced to the first stripping zone 132. The first stripped product stream 134 may be discharged from the first stripping zone 132 may be passed through cyclone separators (not shown) and out of the stripper vessel (not shown). The first stripped product stream 134 may be passed out of the first stripping zone 132. In embodiments, the first stripped product stream 134 may be combined with the first cracking effluent 128. The spent first catalyst 126, which is the first catalyst 124 after stripping out the first stripped product stream 134, may be passed from the first stripping zone 132 to the regeneration zone 162 of the regenerator 160 to be regenerated.

Referring again to FIG. 2, the lesser boiling point fraction 108 may be passed from the feed separator 104 to the second FCC unit 140. Referring again to FIG. 3, the second FCC unit 140 may include a second catalyst/feed mixing zone 156, the second cracking reaction zone 142, a second separation zone 151, and a second stripping zone 152. The lesser boiling point fraction 108 may be introduced to the second catalyst/feed mixing zone 156, where the lesser boiling point fraction 108 may be mixed with the second catalyst 144. During steady state operation of the HSFCC system 150, the second catalyst 144 may include at least the regenerated catalyst 116 that is passed to the second catalyst/feed mixing zone 156 from a second catalyst hopper 174b. In embodiments, the second catalyst 144 may be a mixture of spent second catalyst 146 and regenerated catalyst 116. Alternatively, the second catalyst 144 may be a mixture of spent first catalyst 126 and regenerated catalyst 116. The second catalyst hopper 174b may receive the regenerated catalyst 116 from the regenerator 160 following regeneration of the spent first catalyst 126 and spent second catalyst 146. At initial start-up of the HSFCC system 150, the second catalyst 144 may include fresh catalyst (not shown), which is catalyst that has not been circulated through the first FCC unit 120 or the second FCC unit 140 and the regenerator 160. In embodiments, fresh catalyst may also be introduced to second catalyst hopper 174b during operation of the HSFCC system 150 so that at least a portion of the second catalyst 144 introduced to the second catalyst/feed mixing zone 156 includes the fresh catalyst. Fresh catalyst may be introduced to the second catalyst hopper 174b periodically during operation to replenish lost catalyst or compensate for spent catalyst that becomes permanently deactivated, such as through heavy metal accumulation in the catalyst.

The mixture comprising the lesser boiling point fraction 108 and the second catalyst 144 may be passed from the second catalyst/feed mixing zone 156 to the second cracking reaction zone 142. The mixture of the lesser boiling point fraction 108 and second catalyst 144 may be introduced to a top portion of the second cracking reaction zone 142. The second cracking reaction zone 142 may be a downflow reactor or "downer" reactor in which the reactants flow from the second catalyst/feed mixing zone 156 downward through the second cracking reaction zone 142 to the second separation zone 151. Steam 127 may be introduced to the top portion of the second cracking reaction zone 142 to reduce the hydrocarbon partial pressure of the mixture of the lesser boiling point fraction 108 and the second catalyst 144. The lesser boiling point fraction 108 may be reacted by contact with the second catalyst 144 in the second cracking reaction zone 142 to cause at least a portion of the lesser boiling point fraction 108 to undergo at least one cracking reaction to form at least one cracking reaction product, which may include at least one of the petrochemical products previously described. The second catalyst 144 may have a temperature equal to or greater than the temperature of the second cracking reaction zone 142 and may transfer heat to the lesser boiling point fraction 108 to promote the endothermic cracking reactions.

It should be understood that the second cracking reaction zone 142 of the second FCC unit 140 depicted in FIG. 2 is a simplified schematic of one particular embodiment of the second cracking reaction zone 142, and other configurations of the second cracking reaction zone 142 may be suitable for incorporation into the HSFCC system 150. In embodiments, the second cracking reaction zone 142 may be an up-flow cracking reaction zone. Other cracking reaction zone configurations are contemplated. The second FCC unit may be an HSFCC unit in which, in the second cracking reaction zone 142, the fluidized second catalyst 144 contacts the lesser boiling point fraction 108 at high severity conditions. The temperature of the second cracking reaction zone 142 may be from 500 °C to 800 °C, from 500 °C to 700 °C, from 500 °C to 650 °C, from 500 °C to 600 °C, from 550 °C to 800 °C, from 550 °C to 700 °C, from 550 °C to 650 °C, from 550 °C to 600 °C, from 600 °C to 800 °C, from 600 °C to 700 °C, or from 600 °C to 650 °C. In embodiments, the temperature of the second cracking reaction zone 142 may be from 500 °C to 700 °C. In embodiments, the temperature of the second cracking reaction zone 142 may be from 550 °C to 630 °C. In embodiments, the temperature of the second cracking reaction zone 142 may be different than the temperature of the first cracking reaction zone 122.

A weight ratio of the second catalyst 144 to the lesser boiling point fraction 108 in the second cracking reaction zone 142 (catalyst to hydrocarbon ratio) may be from 5:1 to 40:1, from 5:1 to 35:1, from 5:1 to 30:1, from 5:1 to 25:1, from 5:1 to 15:1, from 5:1 to 10:1, from 10:1 to 40:1, from 10:1 to 35:1, from 10:1 to 30:1, from 10:1 to 25:1, from 10:1 to 15:1, from 15:1 to 40:1, from 15:1 to 35:1, from 15:1 to 30:1, from 15:1 to 25:1, from 25:1 to 40:1, from 25:1 to 35:1, from 25:1 to 30:1, or from 30:1 to 40:1. In embodiments, the weight ratio of the second catalyst 144 to the lesser boiling point fraction 108 in the second cracking reaction zone 142 may be different than the weight ratio of the first catalyst 124 to the greater boiling point fraction 106 in the first cracking reaction zone 122. The residence time of the mixture of second catalyst 144 and the lesser boiling point fraction 108 in the second cracking reaction zone 142 may be from 0.2 seconds (sec) to 3 sec, from 0.2 sec to 2.5 sec, from 0.2 sec to 2 sec, from 0.2 sec to 1.5 sec, from 0.4 sec to 3 sec, from 0.4 sec to 2.5 sec, or from 0.4 sec to 2 sec, from 0.4 sec to 1.5 sec, from 1.5 sec to 3 sec, from 1.5 sec to 2.5 sec, from 1.5 sec to 2 sec, or from 2 sec to 3 sec. In embodiments, the residence time in the second cracking reaction zone 142 may be different than the residence time in the first cracking reaction zone 122.

Following the cracking reaction in the second cracking reaction zone 142, the contents of effluent from the second cracking reaction zone 142 may include second catalyst 144 and the second cracking reaction product stream 148, which may be passed to the second separation zone 151. In the second separation zone 151, the second catalyst 144 may be separated from at least a portion of the second cracking reaction product stream 148. In embodiments, the second separation zone 151 may include one or more gas-solid separators, such as one or more cyclones. The second catalyst 144 exiting from the second separation zone 151 may retain at least a residual portion of the second cracking reaction product stream 148.

After the second separation zone 151, the second catalyst 144 may be passed to the second stripping zone 152, where at least some of the residual portion of the second cracking reaction product stream 148 may be stripped from the second catalyst 144 and recovered as a second stripped product stream 154. Steam may be introduced to the second stripping zone 152 to facilitate stripping the second cracking reaction product 148 from the second catalyst 144. The second stripped product stream 154 may include at least a portion of the steam introduced to the second stripping zone 152 and may be passed out of the second stripping zone 152. The second stripped product stream 154 may pass through cyclone separators (not shown) and out of the stripper vessel (not shown). In embodiments, the second stripped product stream 154 may be combined with the second cracking effluent 148. The spent second catalyst 146, which is the second catalyst 144 after stripping out the second stripped product stream 154, may be passed from the second stripping zone 152 to the regeneration zone 162 of the regenerator 160.

Referring to FIG. 3, the same type of catalyst may be used throughout the HSFCC system 150, such as for the first catalyst 124 and the second catalyst 144. The catalyst (first catalyst 124 and second catalyst 144) used in the HSFCC system 150 may include one or more fluid catalytic cracking catalysts that are suitable for use in the first cracking reaction zone 122 and the second cracking reaction zone 142. The catalyst may be a heat carrier and may provide heat transfer to the greater boiling point fraction 106 in the first cracking reaction zone 122 operated at high severity conditions and the lesser boiling point fraction 108 in the second cracking reaction zone 142 operated at high severity conditions. The catalyst may also have a plurality of catalytically active sites, such as acidic sites for example, that promote the cracking reactions. In embodiments, the catalyst may be a high-activity FCC catalyst having high catalytic activity. Examples of fluid catalytic cracking catalysts suitable for use in the HSFCC system 150 may include, without limitation, zeolites, silica-alumina catalysts, carbon monoxide burning promoter additives, bottoms cracking additives, light olefin-producing additives, other catalyst additives, or combinations of these components. Zeolites that may be used as at least a portion of the catalyst for cracking may include, but are not limited to, Y zeolite, REY zeolites, USY zeolites, RE-USY zeolites, beta zeolites, or combinations of these. The catalyst may also include a shape-selective catalyst additive, such as ZSM-5 zeolite crystals or other pentasil-type catalyst structures, which are often used in other FCC processes to produce light olefins and/or increase FCC gasoline octane. In embodiments, the catalyst may include a mixture of a ZSM-5 zeolite crystals and the cracking catalyst zeolite and matrix structure of a typical FCC cracking catalyst. In embodiments, the catalyst may be a mixture of Y and ZSM-5 zeolite. In embodiments, the catalysts for the HSFCC unit may include the zeolite components in combination with clays, alumina, binders, or combinations of these. In embodiments, the cracking catalysts may be formed into pellets through extrusion, calcining, and sizing.

In embodiments, at least a portion of the catalyst may be modified to include one or more rare earth elements (15 elements of the Lanthanide series of the IUPAC Periodic Table plus scandium and yttrium), alkaline earth metals (Group 2 of the IUPAC Periodic Table), transition metals, phosphorus, fluorine, or any combination of these, which may enhance olefin yield in the first cracking reaction zone 122, second cracking reaction zone 142, or both. Transition metals may include "an element whose atom has a partially filled d sub-shell, or which can give rise to cations with an incomplete d sub-shell" [IUPAC, Compendium of Chemical Terminology, 2nd ed. (the "Gold Book") (1997). Online corrected version: (2006-) "transition element"]. One or more transition metals or metal oxides may also be impregnated onto the catalyst. Metals or metal oxides may include one or more metals from Groups 6-10 of the IUPAC Periodic Table. In embodiments, the metals or metal oxides may include one or more of molybdenum, rhenium, tungsten, or any combination of these. In embodiments, a portion of the catalyst may be impregnated with tungsten oxide.

Referring again to FIGS. 2 and 3, the first FCC unit 120 and the second FCC unit 140 may share the regenerator 160. The regenerator 160 may include a riser 164, the regeneration zone 162, catalyst transfer lines 166 and 168, the first and second catalyst hoppers 174a and 174b, and a flue gas vent 171. The spent first catalyst 126 and the spent second catalyst 146 may be passed to the riser 164 of the regenerator 160, where the spent first catalyst 126 and the spent second catalyst 146 are mixed together with combustion gases 170 and passed to the regeneration zone 162 through the riser 164 to produce the regenerated catalyst 116. The combustion gases 170 and spent first and second catalysts 126 and 146 react in the riser 164 and in the regenerator zone 162 to produce regenerated catalyst 116. The regenerated catalyst 116 collects at the bottom of the regeneration zone 162 and is transferred to the first and second hoppers 174a and 174b. The catalyst transfer lines 166 and 168 are fluidly coupled to the regeneration zone 162 and the first and second catalyst hoppers 174a and 174b, respectively, for passing the regenerated catalyst 116 from the regeneration zone 162 to the first and second catalyst hoppers 174a and 174b. In embodiments, the flue gas vent 171 may be positioned at the top regeneration zone 162 to allow flue gas 172 to exit the regeneration zone 162.

In operation, the spent first catalyst 126 and spent second catalyst 146 may be passed from the first stripping zone 132 and the second stripping zone 152, respectively, to the riser 164 of the regenerator 160. Combustion gas 170 may be introduced to the bottom 168 of the regenerator 160 to mix with the spent first and second catalysts 126 and 146. The combustion gases 170 may include one or more of combustion air, oxygen, fuel gas, fuel oil, other component, or any combinations of these combustion gases. In the riser 164 and the regeneration zone 162, the coke deposited on the spent first catalyst 126 and the spent second catalyst 146 may at least partially oxidize (combust) in the presence of the combustion gases 170 to form at least carbon dioxide and water. In embodiments, the coke deposits on the spent first catalyst 126 and spent second catalyst 146 may be fully oxidized in the regeneration zone 162. Other organic compounds, such as remaining hydrocarbon reactants or reaction products, may also oxidize in the presence of the combustion gases 170 in the regeneration zone. Oxidation of the coke deposits produces heat, which may be transferred to and retained by the regenerated catalyst 116.

The single catalyst regenerator 160 for regenerating the spent first catalyst 126 and the spent second catalyst 146 may improve the overall efficiency of the HSFCC system 150 because the combustion of the coke deposits on the spent first catalyst 126 during catalyst regeneration may produce sufficient heat to raise the temperature of the regenerated catalyst 116 to high severity conditions, and may provide the heat required to conduct the cracking reactions in both the first cracking reaction zone 122 and the second cracking reaction zone 142.

The regenerated catalyst 116 passing out of the regeneration zone 162 may have less than 1 wt. % coke deposits, based on the total weight of the regenerated catalyst 116, such as less than 0.5 wt. %, less than 0.1 wt. %, less than 0.05 wt.% coke deposits, from 0.001 wt.% to 1 wt.%, from 0.001 wt.% to 0.5 wt.%, from 0.001 wt.% to 0.1 wt.%, from 0.001 wt.% to 0.05 wt.%, from 0.005 wt.% to 1 wt.%, from 0.005 wt.% to 0.5 wt.%, from 0.005 wt.% to 0.1 wt.%, from 0.005 wt.% to 0.05 wt.%, from 0.01 wt.% to 1 wt.%, from 0.01 wt.% to 0.5 wt.% to 0.01 wt.% to 0.1 wt.%, from 0.01 wt.% to 0.05 wt.% coke deposits, based on the total weight of the regenerated catalyst 116. Removal of the coke deposits from the catalytically active sites on the catalyst may increase the catalytic activity of the regenerated catalyst 116 compared to the spent first catalyst 126 and spent second catalyst 146. Thus, the regenerated catalyst 116 may have a catalytic activity that is greater than the spent first catalyst 126 and the spent second catalyst 146. The regenerated catalyst 116 may absorb at least a portion of the heat generated from combustion of the coke deposits. The heat may increase the temperature of the regenerated catalyst 116 compared to the temperature of the spent first catalyst 126 and spent second catalyst 146.

As previously discussed, hydrocarbon feed streams 102, such as crude oil for example, can have a wide range of compositions and a wide range of boiling points. The hydrocarbon feed stream 102 may be separated into the greater boiling point fraction 106 and the lesser boiling point fraction 108. The greater boiling point fraction 106 generally has a different composition than the lesser boiling point fraction 108. Thus, each of the greater boiling point fraction 106 and the lesser boiling point fraction 108 may require different operating temperatures and catalyst activities to produce desired yields of one or more petrochemical products or increase the selectivity of the reaction for certain products. For instance, the greater boiling point fraction 106 may be more reactive and, thus, may require less cracking activity than the lesser boiling point fraction 108 to produce sufficient yields of or selectivity for a specific petrochemical product. The lesser cracking activity suitable for the greater boiling point fraction 106 may be provided by reducing the catalytic activity of the first catalyst 124 in the first cracking reaction zone 122, reducing the temperature in the first cracking reaction zone 122, reducing the residence time, reducing the catalyst to oil ratio, or any combinations of these. In contrast, the lesser boiling point fraction 108 may be less reactive and may require greater catalytic activity, such as an increased catalytic activity of the second catalyst 144 in the second cracking reaction zone 142, a temperature in the second cracking reaction zone 142 greater than the temperature of the first cracking zone 122, increased residence time, increased catalyst to oil ratio, or combinations of these, compared to the greater boiling point fraction 106 to produce sufficient yields of or selectivity for the specific petrochemical products.

As previously described in this disclosure, the HSFCC system 150 may include a single regenerator 160 to regenerate the spent first catalyst 126 and the spent second catalyst 146 to produce the regenerated catalyst 116. Therefore, the regenerated catalyst 116 passed to the first FCC unit 120 is the same as and has the same catalytic effectiveness and temperature as the regenerated catalyst 116 passed to the second FCC unit 140. However, as previously discussed, the reaction conditions in the first FCC unit 120 or second FCC unit 140 for producing sufficient yields of or selectivity for specific petrochemical products may be different than the reaction conditions provided by passing the regenerated catalyst 116 to the other of the first FCC unit 120 or the second FCC unit 140.

Referring to FIG. 2, the first cracking effluent 128 and the second cracking effluent 148 may be combined to produce the HSFCC effluent 110. The HSFCC effluent 110 may also include the first stripped product stream 134 (FIG. 3), the second stripped product stream 154 (FIG. 3), or both. The HSFCC effluent 110 may be passed from the HSFCC system 150 to the separation system 200. In embodiments, the first cracking effluent 128, the second cracking effluent 148, the first stripped product stream 134, the second stripped product stream 154, or combinations of these streams may be passed separately and independently to the separation system 200 and combined within the separation system 200.

### Separation System

Referring again to FIG. 1, the system 100 for converting crude oil to greater value chemical products and intermediates includes the separation system 200 disposed downstream of the HSFCC system 150. The HSFCC effluent 110 may be separated into fractions in the separation system 200. The HSFCC effluent 110 may be passed directly from the HSFCC system 150 to the separation system 200 without passing through any intervening reactor or separation system. The separation system 200 may include any suitable separation unit or combinations of separation units, such as, and without limitation, a fractional distillation column, one or more atmospheric distillation column, one or more vacuum distillation columns, or other separation systems operable to separate the HSFCC effluent 110 according to boiling point differences into a plurality of fractions. In embodiments, such as depicted in FIG. 1, the separation system 200 may be operable to separate the HSFCC effluent 110 into at least a light products effluent 202, a mixed C₄ effluent 203, a naphtha effluent 204, and a heavy effluent 205. In embodiments, the separation system 200 may also produce a fuel gas stream 201.

The fuel gas stream 201 may include methane (CH₄) or other one carbon compounds and other non-hydrocarbon gases, such as but not limited to hydrogen, nitrogen, sulfur dioxide, carbon dioxide, carbon monoxide, or other light gases. In embodiments, the fuel gas stream 201 may have at least at least 80%, at least 90%, at least 95%, at least 98%, or at least 99% by weight of the C₁ hydrocarbons from the HSFCC effluent 110.

In embodiments, the light products effluent 202 may comprise C₁-C₃ hydrocarbons, such fuel gases and light olefins (such as ethylene and propylene). The light products effluent 202 may include at least at least 80%, at least 90%, at least 95%, at least 98%, or at least 99% by weight of the C₁-C₃ hydrocarbons from the HSFCC effluent 110. The light products effluent 202 may include at least 80%, at least 90%, at least 95%, at least 98%, or even at least 99% of the constituents of the HSFCC effluent 110 having boiling point temperatures less than or equal to - 20 °C. In embodiments, the light products effluent 202 may include non-hydrocarbon gases, such as but not limited to hydrogen, nitrogen, sulfur dioxide, carbon dioxide, carbon monoxide, or other gases. In embodiments, the separation system 200 may be operable to produce the light products effluent 202 comprising C₂-C₃ hydrocarbons and the fuel gas stream 201 comprising methane and other non-hydrocarbon gases. In embodiments, the light products effluent 202 may include at least at least 80%, at least 90%, at least 95%, at least 98%, or at least 99% by weight of the C₂-C₃ hydrocarbons from the HSFCC effluent 110. The C2-C3 hydrocarbons may include ethane, ethylene, acetylene, propane, propene, or other hydrocarbons having 2 to 3 carbon atoms.

In embodiments, the mixed C₄ effluent 203 may comprise C₄ hydrocarbons, such as but not limited to butane, isobutane, butene, isobutene, butyne, butadiene, and any isomers thereof. The mixed C₄ effluent 203 may include at least at least 80%, at least 90%, at least 95%, at least 98%, or at least 99% by weight of the C₄ hydrocarbons from the HSFCC effluent 110. The mixed C₄ effluent 203 may include at least 80%, at least 90%, at least 95%, at least 98%, or even at least 99% of the constituents of the one or more product streams 110 having boiling point temperatures of from -20 °C to 25 °C.

In embodiments, the naphtha effluent 204 may comprise C₅-C₁₂ hydrocarbons, such as C₅-C₁₂ paraffins. The naphtha effluent 204 may include at least at least 80%, at least 90%, at least 95%, at least 98%, or at least 99% by weight of the C₅-C₁₂ hydrocarbons from the one or more product streams 110. The naphtha effluent 204 may include at least 80%, at least 90%, at least 95%, at least 98%, or even at least 99% of the constituents of the one or more product streams 110 having boiling point temperatures in a range of from greater than 25 °C to 220 °C.

In embodiments, the heavy effluent 205 may comprise C₁₃ or greater hydrocarbons, such as slurry oil. The heavy effluent 205 may include at least at least 80%, at least 90%, at least 95%, at least 98%, or at least 99% by weight of the C₁₃₊ hydrocarbons from the one or more product streams 110. The heavy effluent 205 may include at least 80%, at least 90%, at least 95%, at least 98%, or even at least 99% of the constituents of the one or more product streams 110 having boiling point temperatures greater than or equal to 220 °C. The heavy effluent 205 may include light cycle oil (LCO), heavy cycle oil (HCO), slurry oil, or combinations of these.

### Olefin Separation System

Referring again to FIG. 1, the system 100 for converting crude oil to chemicals may include an olefin separation system 300 disposed downstream of the separation system 200. The light products effluent 202 may be separated into fractions in the olefin separation system 300. The light products effluent 202 may be passed directly from the separation system 200 to the olefin separation system 300 without passing through any intervening reactor or separation system. The olefin separation system 300 may include any suitable separation unit or combination of a plurality of separation units operable to separate C₁-C₃ hydrocarbons into one or more constituent streams. In embodiments, the olefin separation system 300 may include one or more of a cryogenic distillation system, pressure vacuum swing adsorption system, other type of separation system, or any combinations of these separation systems.

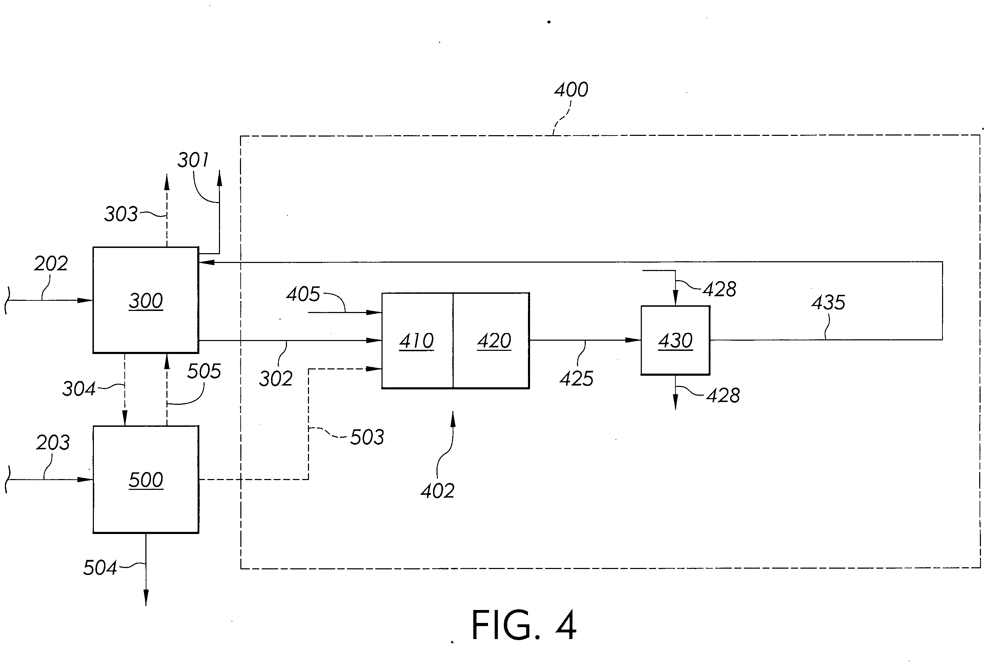

Referring now to FIG. 4, the olefin separation system 300 may be operable to separate the light products effluent 202 into at least a C₂-C₃ olefin effluent 301 and a light saturated hydrocarbon effluent 302. In embodiments, the olefin separation system 300 may be operable to additionally separate a fuel gas effluent 303 from the light products effluent 202. In embodiments, the olefin separation system 300 may be operable to additionally separate a C₄ effluent 304 from the light products effluent 202.

In embodiments, the C₂-C₃ olefin effluent 301 may comprise C₂-C₃ olefins. The light olefins effluent 302 may include at least at least 80%, at least 90%, at least 95%, at least 98%, or at least 99% by weight of the C₂-C₃ olefins, such as but not limited to ethylene and propylene, from the light products effluent 202. In embodiments, the C₂-C₃ olefin effluent 301 may include greater than or equal to 80 wt.%, greater than or equal to 90 wt.%, greater than or equal to 95 wt.%, or greater than or equal to 98% ethylene, propylene, or both based on the total weight of the C₂-C₃ olefin effluent 301 . In embodiments, the light olefins effluent 302 may be passed downstream for further processing or for shipment or use as a product.

The light saturated hydrocarbon effluent 302 may comprise C₂-C₃ saturated hydrocarbons, such as ethane and propane. The light saturated hydrocarbon effluent 302 may include at least at least 80%, at least 90%, at least 95%, at least 98%, or at least 99% by weight of the C₂-C₃ saturated hydrocarbons from the light products effluent 202. In embodiments, the light saturated hydrocarbon effluent 302 may be passed downstream to a pyrolysis cracking system 400 to convert some of the saturated hydrocarbons from the light saturated hydrocarbon effluent 302 to additional ethylene, propylene, or both.

The fuel gas effluent 303 may include methane (CH₄) and any other light non-hydrocarbon gases, such as but not limited to hydrogen, nitrogen, carbon dioxide, carbon monoxide, SO₂, or other non-hydrocarbon gases. The fuel gas effluent 303 may be passed further downstream to a gas plant (not shown), or the fuel gas effluent 303 may be recycled for use as fuel in other processes within the system 100, such as for heat exchangers (not shown) and the like.

The C₄ effluent 304 may include any residual C₄ hydrocarbons passed to the olefin separation system 300 in the light products effluent 202. In embodiments, the C₄ effluent 304 may include any C4 hydrocarbons produced in the pyrolysis cracking system 400 and passed from the pyrolysis cracking system 400 back to the olefin separation system 300. In embodiments, the C₄ effluent 304 may be passed downstream to the C₄ processing system 500.

### Pyrolysis Cracking System

Referring again to FIG. 1, the system 100 for converting crude oil to chemicals may include a pyrolysis cracking system 400 disposed downstream of the olefin separation system 300. At least a portion of the saturated hydrocarbons in the light saturated hydrocarbon effluent 302 may be converted into light olefins in the pyrolysis cracking system 400. The light saturated hydrocarbon effluent 302 may be passed directly from the olefin separation system 300 to the pyrolysis cracking system 400 without passing through any intervening reactor or separation system. In embodiments, a saturated C₄ stream 503 from the C₄ processing system 500 may also be passed to the pyrolysis cracking system 400. The saturated C₄ stream 503 may be combined with the light saturated hydrocarbon effluent 302 upstream of the pyrolysis cracking system 400 or each of the saturated C₄ stream 503 and the light saturated hydrocarbon effluent 302 may be passed separately and independently to the pyrolysis cracking system 400 and combined within the pyrolysis cracking system 400.

Referring again to FIG. 4, a simplified schematic illustration of one particular embodiment of the pyrolysis cracking system 400 is graphically depicted. It should be understood that other configurations of the pyrolysis cracking system may be suitable for incorporation into the system 100 for converting crude oil to light olefins. As depicted in FIG. 4, the pyrolysis cracking system 400 may include one or a plurality of pyrolysis cracking reactors 402. The pyrolysis cracking reactor 402 may include a convection zone 410 and a pyrolysis zone 420 downstream of the convection zone 410. At least the light saturated hydrocarbon effluent 302 and steam 405 may pass into the convection zone 410. In embodiments, the saturated C₄ stream 503 from the C₄ processing system 500 may also be passed to the convection zone 410. In embodiments, the light saturated hydrocarbon effluent 302, steam 405, and a saturated C₄ stream 503 may be passed separately and independently to the pyrolysis cracking reactor 402 and combined within the pyrolysis cracking reactor 402. The flowrate of steam 405 passed into the convection zone 410 may be sufficient to conduct steam pyrolysis in the pyrolysis zone 420 downstream of the convection zone 410. The flowrate of steam 405 into the convection zone 410 may be sufficient to maintain a mass ratio of steam to hydrocarbons in the pyrolysis cracking reactor 402 of from 0.3:1 to 2:1. In the convection zone 410, the light saturated hydrocarbon effluent 302 (and saturated C₄ stream 503, if present) may be preheated to a preheat temperature. The preheat temperature of the convection zone 410 may be from 400 degrees Celsius (°C) to 650 °C.

The constituents present in the convection zone 410 may be passed to the pyrolysis zone 420 downstream of the convection zone 410. In the pyrolysis zone 420, at least the light saturated hydrocarbon effluent 302 may be contacted with the steam 405 at reaction conditions sufficient to cause at least a portion of the hydrocarbons to undergo steam cracking (also known as steam pyrolysis) to produce a pyrolysis zone effluent 425. The pyrolysis zone 420 may operate at a temperature of from 700 °C to 900 °C. The pyrolysis zone 420 may operate with a residence time of from 0.05 seconds to 2 seconds, where the residence time is the duration of time that the hydrocarbons are in contact with the steam at the reaction temperature of from 700 °C to 900 °C. The mass ratio of steam 405 to hydrocarbons in the pyrolysis zone 420 may be from 0.3:1 to 2:1.

The pyrolysis zone effluent 425 may exit the pyrolysis zone 420 of the pyrolysis cracking reactor 402 and may be passed through a heat exchanger 430 downstream of the pyrolysis cracking reactor 402. In the heat exchanger 430, a process fluid 428, such as water, pyrolysis fuel oil, or other process stream, may cool the pyrolysis zone effluent 425 to form the cracking reaction effluent 435. The cracking reaction effluent 435 may include a mixture of cracked hydrocarbon-based materials which may be separated into one or more petrochemical products included in one or more system product streams. For example, the cracking reaction effluent 435 may include ethylene, propylene, or both. In embodiments, the cracking reaction effluent 435 may also include other olefins, such as but not limited mixed butenes (1-butene, *trans*-2-butene, *cis*-2-butene, isobutene, or combinations of these), 1,3-butadiene, C5+ olefins, or combinations of these; light gases, such as but not limited to methane, hydrogen, and steam; saturated hydrocarbons, such as but not limited to ethane, propane, butane, isobutane, C5+ alkanes, or combinations of these. The cracking reaction effluent 435 may be passed back to the olefin separation system 300 to separate the light olefins from the light alkanes and C₄₊ constituents to produce at least the C₂-C₃ olefin effluent 301 and the light saturated hydrocarbon effluent 302. The light alkanes and C₄₊ constituents may be sent back to the pyrolysis cracking system 400 as a portion of the light saturated hydrocarbon effluent 302 to be converted into light olefins.

### C4 Processing System

Referring again to FIG. 1, the system 100 for converting crude oil to chemicals may include a C₄ processing system 500 disposed downstream of the separation system 200. The mixed C₄ effluent 203 may be converted into light olefins in the C₄ processing system 500. The mixed C₄ effluent 203 may be passed directly from the separation system 200 to the C₄ processing system 500 without passing through any intervening reactor or separation system.

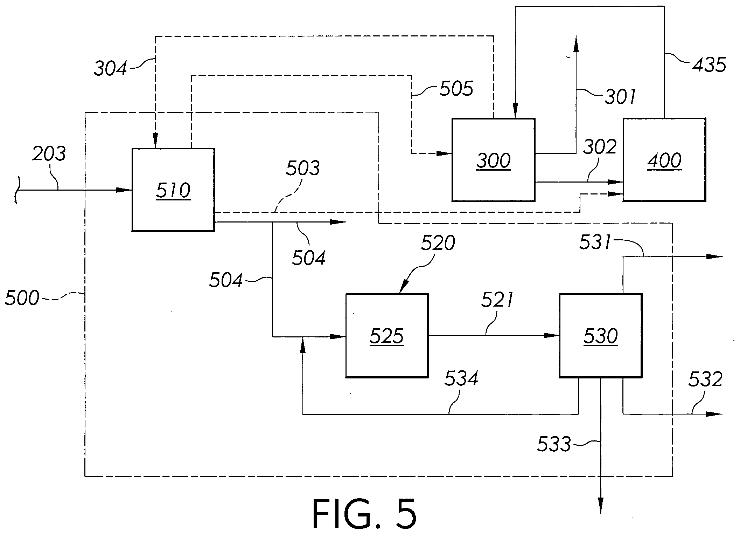

Referring now to FIG. 5, a simplified schematic illustration of one embodiment of the C₄ processing system 500 is schematically depicted. The C₄ processing system 500 may include a C₄ separation unit 510, a metathesis reactor 520 downstream of the C₄ separation unit 510, and a metathesis effluent separation unit 530 downstream of the metathesis reactor 520. It should be understood that other configurations of the C₄ processing system may be suitable for incorporation into the system 100 for converting crude oil to light olefins.

As depicted in FIG. 5, the C₄ processing system 500 may include the C₄ separation unit 510, which may include one or a plurality of separation units. The C₄ separation unit 510 may include any suitable separation unit or combination of separation units, such as, and without limitation, distillation columns, solvent extraction devices, or combinations thereof. The C₄ separation unit 510 may be operable to separate the mixed C₄ effluent 203 into a saturated C₄ stream 503 and a C₄ olefin stream 504. It is contemplated that some C₂-C₃ hydrocarbons may remain in the mixed C₄ effluent 203 and may be separated from the C₄ effluent 203 in the C₄ separation unit 510 to produce a C₂-C₃ stream 505. In embodiments, any remaining C₂-C₃ hydrocarbons in the C₂-C₃ stream 505 may be passed to the olefin separation system 300 to be processed. Similarly, in embodiments, it is contemplated that some C₄ hydrocarbons may remain in the light products effluent 202. In embodiments, any C₄ hydrocarbons recovered from the olefin separation system 300 may be passed to the C₄ separation unit 510 as the C₄ effluent 304. Likewise, any C₂-C₃ hydrocarbons separated in the C₄ separation unit 510 may be passed to the olefin separation system 300 as a C₂-C₃ effluent 505. The saturated C₄ stream 503 may comprise saturated C₄ hydrocarbons such as butane and iso-butane. The saturated C₄ stream 503 may be passed to the pyrolysis cracking system 400 to convert the saturated C₄ hydrocarbons to light olefins through steam cracking, as previously described in the present disclosure. The C₄ olefin stream 504 may comprise normal butenes, iso-butene, and other unsaturated C₄ hydrocarbons.

Referring again to FIG. 5, in embodiments, the C₄ olefin stream 504 may be passed to at least one metathesis reactor 520 downstream of the C₄ separation unit 510 for conversion to C₂-C₃ olefins through one or more metathesis reactions. The metathesis reactor 520 may be operable to produce a product stream 521 comprising propene, ethylene, or both from the C₄ olefin stream 504 (comprising butenes). The metathesis reactor 520 may generally include a metathesis reaction zone 525 comprising at least one metathesis catalyst. Optionally, the metathesis reactor 520 may further include one or more additional reaction zones, such as isomerization reaction zones, additional metathesis reaction zones, or cracking reaction zones, which are not depicted in FIG. 5. Generally, the metathesis reaction zones, including the metathesis reaction zone 525, may be positioned downstream of the isomerization reaction zones, and the cracking reaction zones may be positioned downstream of the metathesis reaction zones. As depicted in FIG. 5, the metathesis reactor 520 may comprise the metathesis reaction zone 525 disposed within the metathesis reactor 520. While not depicted, it should be understood that any additional reaction zones may also be disposed within the metathesis reactor 520, such as in a series of catalyst beds. Alternatively, each reaction zone, including the metathesis reaction zone 525, may be disposed within independent reactors arranged in series. For example, the C₄ processing system 500 may comprise three reactors arranged in series where the first reactor comprises an isomerization reaction zone, the second reactor comprises a metathesis reaction zone, and the third reactor comprises a cracking reaction zone. The isomerization effluent of the first reactor may enter the second reactor as an inlet stream, the metathesis effluent of the second reactor may enter the third reactor as an inlet stream, and the cracking effluent of the third reactor may exit the C₄ processing system 500 as a product stream.

Referring again to FIG. 5, the C₄ olefin stream 504 may be introduced to the metathesis reactor 520. As mentioned previously in the present disclosure, the C₄ olefin stream 504 may generally comprise butenes, such as 1-butene, *trans*-2-butene, *cis*-2-butene, or combinations of one or more of these isomers. The C₄ olefin stream 504 may comprise from 0 (zero) weight percent (wt.%) to 100 wt.% 1-butene, based on the total weight of the C₄ olefin stream 504. For example, the C₄ olefin stream 504 may comprise from 0 wt.% to 75 wt.%, from 0 wt.% to 50 wt.%, from 0 wt.% to 25 wt.%, from 25 wt.% to 100 wt.%, from 25 wt.% to 75 wt.%, from 25 wt.% to 50 wt.%, from 50 wt.% to 100 wt.%, from 50 wt.% to 75 wt.%, or from 75 wt.% to 100 wt.% 1-butene, based on the total weight of the C₄ olefin stream 504. The C₄ olefin stream 504 may comprise from 0 weight percent (wt.%) to 100 wt.% 2-butene, based on the total weight of the C₄ olefin stream 504. For example, the C₄ olefin stream 504 may comprise from 0 wt.% to 75 wt.%, from 0 wt.% to 50 wt.%, from 0 wt.% to 25 wt.%, from 25 wt.% to 100 wt.%, from 25 wt.% to 75 wt.%, from 25 wt.% to 50 wt.%, from 50 wt.% to 100 wt.%, from 50 wt.% to 75 wt.%, or from 75 wt.% to 100 wt.% 2-butene, based on the total weight of the C₄ olefin stream 504. In embodiments, the C₄ olefin stream 504 may also include isobutene. In embodiments, the C4 olefin stream 504 may be treated to remove isobutene upstream of the metathesis reactor 520.

Accordingly, the C₄ olefin stream 504 may comprise sufficient amounts of butene for the production of propene. In embodiments, the C₄ olefin stream 504 may be subjected to hydrogenation to remove butadienes. In embodiments, the C₄ olefin stream 504 may be subjected to a process to remove isobutene, such as an MTBE process.

Referring still to FIG. 5, the C₄ olefin stream 504 may be introduced to the metathesis reactor 520 and contacted with a metathesis catalyst in the metathesis reaction zone 525 to produce a metathesis reaction effluent 521. The C₄ olefin stream 504 may be passed to the metathesis reactor 520. The metathesis reactor 520 may be in fluid communication with the C₄ separation unit 510 and may receive a portion of the C₄ olefin stream 504 from the C₄ separation unit 510. The C₄ olefin stream 504 may be passed directly from the C₄ separation unit 510 to the metathesis reactor 520 without passing through any intervening reactor or separation system. The metathesis reactor 520 may be operable contact the C₄ olefin stream 504 with a metathesis catalyst under operating conditions sufficient to produce the metathesis reaction effluent 521. In embodiments, an ethylene stream (not shown) may be passed to the metathesis reactor 520, which may facilitate cross-metathesis reaction between 2-butenes and the ethylene to produce propylene. In embodiments, ethylene may be formed from self-metathesis of 1-butene and then reacted with 2-butenes to produce propylene. Other metathesis reaction schemes are contemplated.

The metathesis catalyst may generally comprise a catalytically active compound capable of promoting the self-metathesis of butenes, cross-metathesis of butenes with other olefins, or a combination of both. The other olefins may include ethylene, propylene, or other olefins produced in the metathesis reaction zone 525 through self-metathesis or olefins, such as ethylene, added to the metathesis reactor 520 as a separate reactant stream. The morphology, type, and amount of the catalytically active compound may determine the catalytic activity of the catalyst. The catalytically active compound may be a metal or metal oxide, such as one or more oxides of a metal from Groups 6-10 of the International Union of Pure and Applied Chemistry (IUPAC) Periodic Table, such as an oxide of molybdenum, rhenium, tungsten, manganese, titanium, cerium, or combination of these. For example, the catalytically active compound may be tungsten oxide. As noted previously in the present disclosure, the catalytically active compound of the metathesis catalyst may be supported by (that is, deposited on) the catalyst support material. The catalytically active compound of the metathesis catalyst may be dispersed throughout the catalyst support material, deposited on the surface of the catalysts support material, or combinations of these. Generally, at least a portion of the catalytically active compound may be accessible at the surfaces of the catalyst support material, such as at the outer and pore surfaces of the catalyst support material.

The metathesis catalyst may comprise the catalytically active compound in an amount sufficient to improve the reaction rate of the reactions catalyzed by the metathesis catalysts. The metathesis catalyst may comprise from 1 wt.% to 20 wt.% catalytically active compound, based on the total weight of the metathesis catalyst. For example, metathesis catalyst 112 may comprise from 1 wt.% to 16 wt.%, from 1 wt.% to 12 wt.%, from 1 wt.% to 8 wt.%, from 1 wt.% to 4 wt.%, from 4 wt.% to 20 wt.%, from 4 wt.% to 16 wt.%, from 4 wt.% to 12 wt.%, from 4 wt.% to 8 wt.%, from 8 wt.% to 20 wt.%, from 8 wt.% to 16 wt.%, from 8 wt.% to 12 wt.%, from 12 wt.% to 20 wt.%, from 12 wt.% to 16 wt.%, or from 16 wt.% to 20 wt.% catalytically active compound, based on the total weight of the metathesis catalyst.

Referring still to FIG. 5, the C₄ olefin stream 504 may be contacted with the metathesis catalyst in the metathesis reaction zone 525 of the metathesis reactor 520 at a temperature sufficient to promote the production of propylene from the C₄ olefins in the C₄ olefin stream 504. The operational temperature may be from 400 °C to 600 °C. For example, the operational temperature may be from 400 °C to 575 °C, from 400 °C to 550 °C, from 400 °C to 525 °C, from 400 °C to 500 °C, from 400 °C to 475 °C, from 400 °C to 450 °C, from 400 °C to 425 °C, from 425 °C to 600 °C, from 425 °C to 575 °C, from 425 °C to 550 °C, from 425 °C to 525 °C, from 425 °C to 500 °C, from 425 °C to 475 °C, from 425 °C to 450 °C, from 450 °C to 600 °C, from 450 °C to 575 °C, from 450 °C to 550 °C, from 450 °C to 525 °C, from 450 °C to 500 °C, from 450 °C to 475 °C, from 475 °C to 600 °C, from 475 °C to 575 °C, from 475 °C to 550 °C, from 475 °C to 525 °C, from 475 °C to 500 °C, from 500 °C to 600 °C, from 500 °C to 575 °C, from 500 °C to 550 °C, from 500 °C to 525 °C, from 525 °C to 600 °C, from 525 °C to 575 °C, from 525 °C to 550 °C, from 550 °C to 600 °C, from 550 °C to 575 °C, or from 575 °C to 600 °C.

The C₄ processing system 500 may also include the metathesis effluent separation unit 530 downstream of the metathesis reactor 520. The metathesis effluent separation unit 530 may include any suitable separation unit, such as, and without limitation, distillation columns, solvent extraction devices, or combinations of these separation units. In embodiments, the metathesis reaction effluent 521 may be passed from the metathesis reactor 520 to the metathesis effluent separation unit 530, which may be operable to separate the metathesis reaction effluent 521 into an ethylene stream 531, a propylene stream 532, a C₅₊ stream 533, and a C₄ recycle stream 534. The C₄ recycle stream 534 may comprise any unconverted butenes from the C₄ olefin stream 504. The C₄ olefin stream 504 and the C₄ recycle stream 534 may be mixed prior to entering the metathesis reactor 520 or they may be introduced to the metathesis reactor 520 separately.

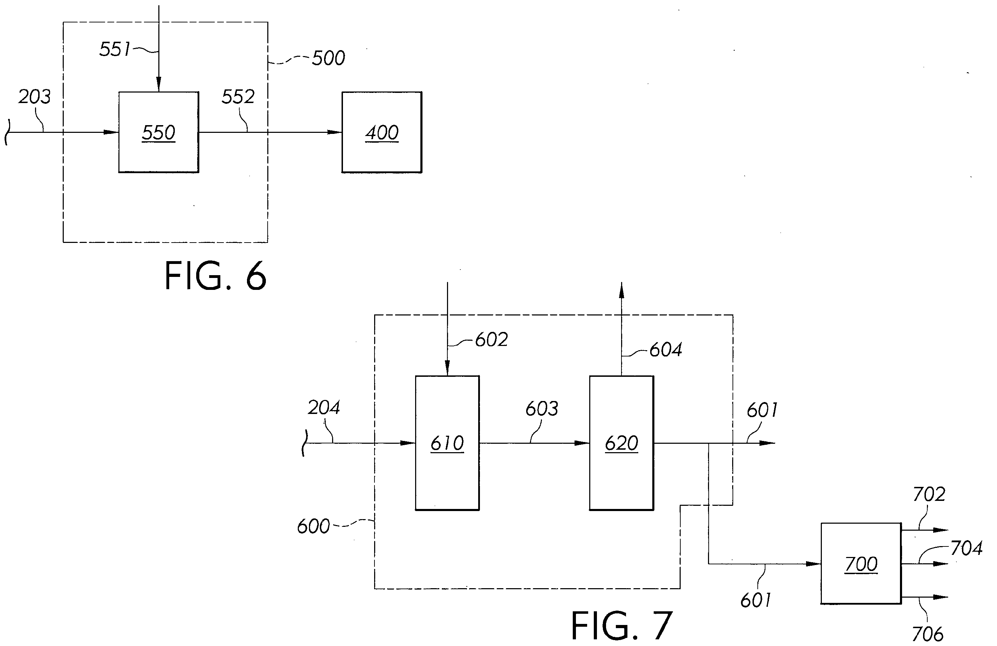

Referring now to FIG. 6, a simplified schematic illustration of another embodiment within the C₄ processing system 500 is graphically depicted. As depicted in FIG. 6, the C₄ processing system 500 may include a hydrogenation reactor 550. In embodiments, the C₄ olefin stream 504 and hydrogen 551 may be passed into a hydrogenation reactor 550 and contacted in the presence of a hydrogenation catalyst to produce a hydrogenation reactor effluent 552. The hydrogenation reactor effluent 552 may comprise one or more saturated C₄ hydrocarbons, such as butane, isobutane, or a combination of butane and isobutane. As depicted in FIG. 6, the C₄ olefin stream 504 may be passed to the hydrogenation reactor 550. The reaction of the C₄ olefin stream 504 and hydrogen 551 may be performed with a hydrogenation catalyst, such as, but not limited to, cobalt-molybdenum (Co-Mo), nickel-molybdenum (Ni-Mo), nickel-tungsten (Ni-W), and/or noble metal catalysts. In embodiments, the catalyst may be supported by alumina. Without being bound by any theory, it is believed that sulfide catalysts such as cobalt-molybdenum (Co-Mo) nickel-molybdenum (Ni-Mo) may be utilized because they may contain active metal sites, which are capable of promoting the hydrogenation reactions and may outperform other catalysts. The hydrogenation reactor effluent 552 may be passed to the pyrolysis cracking system 400 to convert the saturated C₄ hydrocarbons to light olefins through steam cracking, as described above. It should be understood that embodiments depicted within FIG. 5 and FIG. 6 may be combined as an embodiment that includes the metathesis reactor 520, the separation unit 530, and the hydrogenation reactor 550.

### Naphtha Reforming System

Referring again to FIG. 1, the system 100 for converting crude oil to chemicals may include a naphtha reforming system 600 disposed downstream of the separation system 200. The naphtha effluent 204 may be passed to the naphtha reforming system 600, where the naphtha effluent 204 may be hydrotreated and reformed. The naphtha effluent 204 may be passed directly from the separation system 200 to the naphtha reforming system 600 without passing through any intervening reactor or separation system.

Referring now to FIG. 7, a simplified schematic illustration of one embodiment of the naphtha reforming system 600 is depicted. It should be understood that other configurations of the naphtha reforming system 600 may be suitable for incorporation into the system 100 for converting crude oil to light olefins. As depicted in FIG. 7, the naphtha reforming system 600 may include one or a plurality of naphtha hydrotreaters 610.

The naphtha effluent 204 and hydrogen 602 may be passed to the naphtha hydrotreater 610. The naphtha hydrotreater 610 may comprise at least one hydrotreating catalyst. In embodiments, the naphtha hydrotreater 610 may include a plurality of different hydrotreating catalysts disposed in different catalyst beds arranged in series. It is contemplated that the hydrotreating catalysts may be cobalt-molybdenum (Co-Mo), nickel-molybdenum (Ni-Mo), nickel-tungsten (Ni-W), and/or noble metal catalysts. In embodiments, the hydrotreating catalyst may be supported on a catalyst support, such as an alumina support. The naphtha hydrotreater 610 may be operable to reduce the content of metals, sulfur, nitrogen, or combinations of these constituents in the naphtha effluent 204 to produce a hydrotreated effluent 603 that includes hydrotreated naphtha. For example, the hydrotreated effluent 603 passed out of the naphtha hydrotreater 610 may have concentrations of one or more impurities (e.g., sulfur, metals, nitrogen, or combinations of these constituents) that is less than the concentrations of the one or more impurities in the naphtha effluent 204 by at least 25%, at least 50%, or even at least 75%.

The naphtha reforming system 600 may further include a naphtha reforming unit 620. The hydrotreated effluent 603 may be passed to the naphtha reforming unit 620. The naphtha reforming unit 620 may be in fluid communication with the naphtha hydrotreater 610 and may receive the hydrotreated effluent 603 from the naphtha hydrotreater 610. The hydrotreated effluent 603 may be passed directly from the naphtha hydrotreater 610 to the naphtha reforming unit 620 without passing through any intervening reactor or separation system. The naphtha reforming unit 620 may be operable to reform the hydrotreated effluent 603 to produce a reformate 601.

The hydrotreated effluent 603 may be passed to the naphtha reforming unit 620 to upgrade the hydrotreated effluent 603 to improve its quality, such as by increasing the octane number to produce the reformate 601 that can be used as a gasoline blending stream or feedstock for an aromatic recovery complex 700. The naphtha reforming unit 620 may be a catalytic reforming process. In catalytic reforming processes, paraffins, naphthenes, and other C₅₊ hydrocarbons can be restructured to produce isomerized paraffins and aromatics of relatively higher octane numbers. In embodiments, the naphtha reforming unit 620 may produce additional light aromatic compounds, which may include benzene, toluene, xylenes, ethylbenzene, or combinations of these light aromatic compounds. Catalytic reforming can convert low octane n-paraffins to i-paraffins and naphthenes.

The chemical reactions involved in catalytic reforming can be grouped into four categories, which include cracking, dehydrocyclization, dehydrogenation, and isomerization. A particular hydrocarbon molecule of the hydrotreated effluent 603 may undergo one or more than one category of reaction during the reforming process to form one or a plurality of different molecules or products.

The naphtha reforming unit 620 may contact the hydrotreated effluent 603 with a reforming catalyst under operating conditions sufficient to cause at least a portion of the hydrotreated effluent 603 to undergo one or more reactions to produce a reforming effluent, which may then be separated into the reformate 601 and a hydrogen effluent 604. The naphtha reforming unit 50 may be operated at a temperature of from 400 °C to 560 °C, or from 450 °C to 560 °C. The naphtha reforming unit 620 may be operated at a pressure of from 100 kilopascals (kPa) to 5,000 kPa (from 1 bar to 50 bar), or from 100 kPa to 2,000 kPa (from 1 bar to 20 bar). The naphtha reforming unit 620 may be operated at a liquid hourly space velocity (LHSV) of from 0.5 per hour (hr⁻¹) to 4 h⁻¹, or from 0.5 h⁻¹ to 2 h⁻¹.

The reforming catalysts for catalytic reforming processes in the naphtha reforming unit 620 can be either mono-functional or bi-functional reforming catalysts, which can contain precious metals, such as one or more metals from Groups 8-10 of the IUPAC periodic table, as active components (Group VIIIB in the Chemical Abstracts Services (CAS) system). The metals may be supported on a catalyst support, such as but not limited to an alumina, silica, titania, or combination of these supports. The reforming catalyst can be a bi-functional catalyst that has both metal sites and acidic sites. The reforming catalyst may be a platinum or palladium supported on an alumina support. The composition of the hydrotreated effluent 603, the impurities present in the hydrotreated effluent 603, and the desired products in the naphtha reformate 603 may influence the selection of reforming catalyst, reforming process type, and operating conditions. Types of chemical reactions can be targeted by a selection of catalyst or operating conditions known to those of ordinary skill in the art to influence both the yield and selectivity of conversion of paraffinic and naphthenic hydrocarbon precursors to particular aromatic hydrocarbon structures.

The naphtha reforming unit 620 may be any one of several types of catalytic reforming process configurations, which differ in the manner in which they regenerate the reforming catalyst to remove the coke formed during the reforming process. Catalyst regeneration, which involves combusting detrimental coke in the presence of oxygen, can include a semi-regenerative process, a cyclic regeneration process, or continuous regeneration process. During semi-regeneration, the entire unit, including all reactors in the series, are shut-down for catalyst regeneration in all reactors. Cyclic configurations utilize an additional "swing" reactor to permit one reactor at a time to be taken off-line for regeneration while the others remain in service. Continuous catalyst regeneration configurations, which are the most complex, provide for continuous operation by catalyst removal, regeneration and replacement. While continuous catalyst regeneration configurations may enable the severity of the operating conditions to be increased due to higher catalyst activity, the associated capital investment is necessarily higher.

As mentioned above, the naphtha reforming unit 620 may also produce a separate hydrogen effluent 604. The naphtha reforming unit 620 may include a reformed effluent separation system (not shown) that may be operable to separate an effluent from the reforming reactor into the reformate 601 and the hydrogen effluent 604. The hydrogen effluent 604 may be recovered or may be recycled back to one or more of the naphtha hydrotreater 610 as at least a portion of the hydrogen streams to that unit. The reformate 601 may be passed to the aromatic recovery complex 700.

### Aromatic Recovery Complex

Referring again to FIG. 1, the system 100 may include an aromatic recovery complex 700 disposed downstream of the naphtha reforming system 600. The aromatic recovery complex 700 may be in fluid communication with naphtha reforming unit 620 and may receive all or at least a portion of the reformate 601 from the naphtha reforming system 600. The aromatic recovery complex 700 may process the reformate 601 to produce at least one aromatic product effluent 702, a gasoline pool stream 704, and an aromatic bottoms stream 706. The aromatic recovery complex 700 may be operable to separate the reformate 601 into the at least one aromatic product effluent 702, a gasoline pool stream 704, and the aromatic bottoms stream 706. The aromatic recovery complex 700 may also be operable to convert one or more aromatic compounds in the reformate 601 to other aromatic compounds, such as xylenes or other gasoline pool components.

In the aromatic recovery complex 700, the reformate 601 may be subjected to several processing steps to recover greater value products, such as mixed xylenes and benzene, and to convert lower value products, such as toluene, into greater value products. For example, the aromatic compounds present in the reformate 601 can be separated into different fractions by carbon number, such as but not limited to a C₅ fraction, a C₆ fraction comprising benzene, a C₇ fraction comprising toluene, a C₈ fraction including xylenes and ethylbenzene, and a C₉₊ fraction (aromatic bottoms stream 706). The C₈ fraction may be subjected to one or more operations to convert ethylbenzene, ortho-xylene, and meta-xylene to produce greater yield of para-xylene, which is of greater value. Para-xylene can be recovered in high purity from the C₈ fraction by separating the *para*-xylene from the *ortho*-xylene, *meta*-xylene, and ethylbenzene using selective adsorption or crystallization. The *ortho*-xylene and *meta*-xylene remaining from the *para*-xylene separation can be isomerized to produce an equilibrium mixture of xylenes. The ethylbenzene can be isomerized into xylenes or can be dealkylated to benzene and ethane. The *para*-xylene can then be separated from the *ortho*-xylene and the *meta*-xylene using adsorption or crystallization, and the *para*-xylene-depleted-stream can be recycled to extinction to the isomerization unit and then to the *para*-xylene recovery unit until all of the *ortho*-xylene and *meta*-xylene are converted to *para*-xylene and recovered.

Toluene may be recovered as a separate fraction, such as a C₇ fraction, and then can be converted into greater value products, such as but not limited to benzene or xylenes. One toluene conversion process can include the disproportionation of toluene to make benzene and xylenes. Another toluene conversion process can include the hydrodealkylation of toluene to make benzene. Another toluene conversion process can include the transalkylation of toluene to make benzene and xylenes. Both toluene disproportionation and toluene hydrodealkylation can result in the formation of benzene.

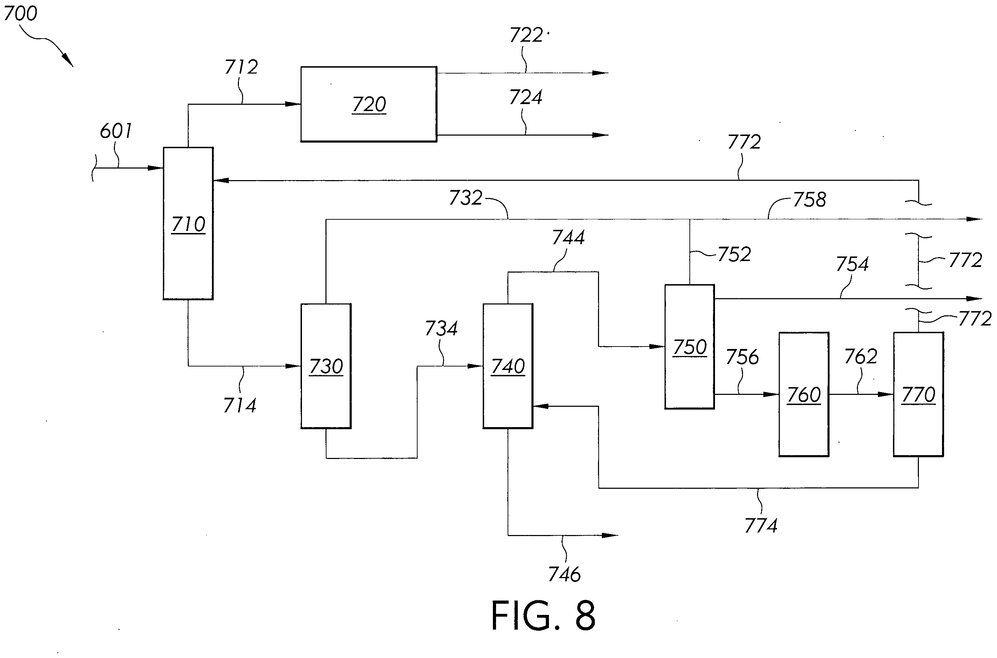

Referring to FIG. 8, an embodiment of the aromatic recovery complex 700 is schematically depicted. The reformate 601 from the naphtha reforming unit 620 (FIG. 7) may be passed to a reformate splitter 710 that may separate the reformate 601 into two fractions: a light reformate stream 712 comprising C₅-C₆ hydrocarbons, and a heavy reformate stream 714 comprising C₇₊ hydrocarbons. In embodiments, the reformate 601 may be hydrotreated (not shown) prior to being passed to the aromatic recovery complex 700. Hydrotreating the reformate 601 may remove mono-olefins, diolefins, or both before the reformate 601 is passed to the aromatic recovery complex 700. The light reformate stream 712 may be passed to an extraction unit 720, which may extract the benzene as benzene product in benzene stream 724 and recover substantially benzene-free gasoline in raffinate motor gasoline (mogas) stream 722. The mogas stream 722 may include at least some C₅₊ raffinate. At least a portion of the mogas stream 722 may be passed upstream to the pyrolysis cracking system for conversion to light olefins. The heavy reformate stream 714 may be passed to a splitter 730 which may separate the heavy reformate stream 714 to produce a first C₇ mogas stream 732 and a C₈₊ hydrocarbon stream 734. The C₈₊ hydrocarbon stream 734 may be passed to a clay tower (not shown) to remove olefin compounds from the C₈₊ hydrocarbon stream 734.

Still referring to FIG. 8, the C₈₊ hydrocarbon stream 734 may be passed to a xylene rerun unit 740, which may separate the C₈₊ hydrocarbon stream 734 into a C₈ hydrocarbon stream 744 and the aromatic bottoms stream 746, which is a C₉₊ hydrocarbon stream comprising C₉₊ hydrocarbons. C₈ hydrocarbon stream 744 may be passed to a *para*-xylene recovery unit 750 that may recover *para*-xylene as *para*-xylene product stream 754. The *para*-xylene recovery unit 750 may also produce a second C₇ cut mogas stream 752, which may be combined with the first C₇ cut mogas stream 732 from splitter 730 to produce C₇ cut mogas stream 758, which may include toluene. The at least one aromatic product effluent 702 in FIGS. 1 and 7, may include the C7 cut mogas stream 758, which may include toluene. Referring again to FIG. 8, other xylenes (*meta-*xylene, *ortho*-xylene, and any trace *para*-xylene not passed out of the *para*-xylene recovery unit 750 in the *para*-xylene product stream 754) may be recovered and passed to a xylene isomerization unit 760 through mixed xylene stream 756. The xylene isomerization unit 760 may isomerize at least a portion of *ortho*-xylene, *meta*-xylene, or both, in the mixed xylene stream 756 to *para-*xylene. The isomerization effluent 762 may be passed from the xylene isomerization unit 760 to a splitter column 770, which may separate the isomerization effluent 762 into a splitter top stream 772 and a splitter bottoms stream 774. The splitter bottoms stream 774 may include the *para-*xylene produced in the xylene isomerization unit 760 as well as the remaining *ortho*-xylene and *meta*-xylene. The splitter bottoms stream 774 may be passed back to the xylene rerun unit 740 so that the xylenes can be separated and passed to the *para*-xylene recovery unit 750 for further recovery of *para*-xylene. The splitter top stream 772 may be recycled back to reformate splitter 710.

The raffinate mogas stream 722 may be passed out of the aromatic recovery complex 700 as the gasoline pool stream 704 (FIGS. 1 and 7), which may be passed to the gasoline pool for blending into fuels. In embodiments, the gasoline pool stream 704 (FIGS. 1 and 7), including the raffinate mogas stream 722, may be passed to the pyrolysis cracking system 400 for conversion to light olefins. The gasoline pool stream 704 comprising the raffinate mogas stream 722 may have less than or equal to 3 volume percent benzene, or less than or equal to 1 volume percent benzene. The at least one aromatic stream 702 (FIGS. 1 and 7) passed out of the aromatic recovery complex 700 may include one or more of the benzene stream 724, the *para*-xylene product stream 754, the C₇ cut mogas stream 758, or combinations of these. The aromatic bottoms stream 706 may include the C₉₊ aromatic compounds from the xylene rerun unit 740 of the aromatic recovery complex 700. The aromatic bottoms stream 706 (FIGS. 1 and 7) may include the heavier fraction, such as C₉₊ alkylated mono-aromatics, and may be a more complex mixture of compounds including di-aromatics. The aromatic bottoms stream 706 may include C₉₊ aromatic compounds having an atmospheric boiling point temperature in a range of from 150 °C to 350 °C. Since olefins are detrimental in the extraction / adsorption process within the aromatic recovery complex 700, olefin compounds can be removed using a clay tower or selective hydrogenation. As previously discussed, the C₈₊ hydrocarbon stream 734 from the splitter 730 may be passed to a clay tower (not shown) to remove olefin compounds from the C₈₊ hydrocarbon stream 734. Due to the acidic nature of the clays, olefinic aromatics such as styrene can react with other aromatic molecule via an alkylation reaction to form bridged di-aromatic molecules. These di-aromatic molecules can end up in the aromatic bottoms stream 706.

### Heavy Oil Processing System

Referring again to FIG. 1, the system 100 for converting crude oil to chemicals may include an heavy oil processing system 800 disposed downstream of the separation system 200. The heavy effluent 205 may be separated into fractions in the heavy oil processing system 800. The heavy effluent 205 may be passed directly from the separation system 200 to the heavy oil processing system 800 without passing through any intervening reactor or separation system.

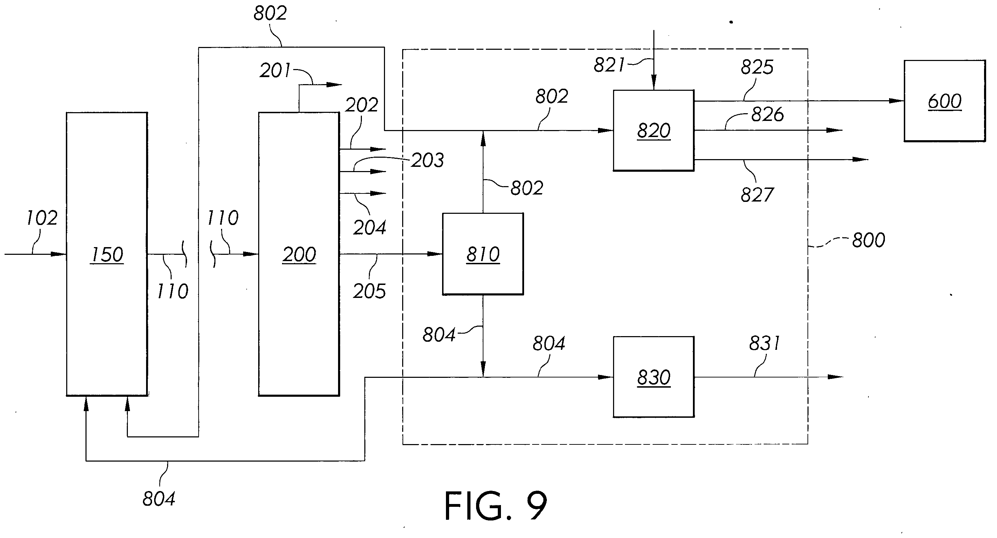

Now referring to FIG. 9, the heavy oil processing system 800 may comprise a separation unit 810, a light cycle oil (LCO) hydrocracking unit 820, and a heavy oil treatment unit 830. In embodiments, the heavy effluent 205 may be separated in the separation unit 810 into at least a light cycle oil 802 and a heavy cycle oil 804. In embodiments, at least a portion of the light cycle oil 802 may be passed upstream to the HSFCC system 150 for heat balance. In embodiments, at least a portion of the heavy cycle oil 804 may be passed upstream to the HSFCC system 150 for heat balance. In embodiments, at least a portion of the light cycle oil 802, at least a portion of the heavy cycle oil 804, may be recycled back to the HSFCC system 150 for further processing.

At least a portion of the light cycle oil 802 and hydrogen 821 may be passed to the LCO hydrocracking unit 820. The LCO hydrocracking unit 820 may comprise at least one hydrocracking catalyst. The LCO hydrocracking unit 820 may be operable to contact the light cycle oil 802 with the hydrogen 821 in the presence of the at least one hydrocracking catalyst, which may cause at least a portion of the light cycle oil 802 to undergo cracking to produce a naphtha stream 825, a diesel stream 826, a fuel oil stream 827, or a combination of these. In embodiments, the naphtha stream 825 may be passed to the naphtha reforming system 600 to produce the reformate effluent, as described above. In embodiments, at least a portion of the heavy cycle oil may be passed to the heavy oil treatment unit 830 to process the heavy cycle oil 804 to produce at least fuel oil 831.

### EXAMPLES

The following non-limiting examples illustrate one or more features of the present disclosure. The examples are illustrative in nature, and should not be understood to limit the subject matter of the present disclosure.

Referring to FIGS. 1 and 2, various configurations of the system 100 are evaluated for economic performance. In the following Examples, experimental data for the HSFCC system 150 having the feed separator 104, the first FCC unit 120, and the second FCC unit 140 was generated using an experimental scale reaction system. The experimental data comprising the composition of the HSFCC effluent 110 obtained from the HSFCC system 150 is then used to model multiple configurations of the downstream unit operations of the system 100 for separating and converting the HSFCC effluent 110 from the HSFCC unit to greater value chemical products and intermediates.

The experimental data for the composition of the HSFCC effluent was prepared by separating a crude oil into a greater boiling point fraction and a lesser boiling point fraction. The crude oil was AXL crude oil having the composition provided in Table 2. Each of the greater boiling point fraction and the lesser boiling point fraction were then subjected to catalytic cracking in a fixed-bed micro-activity cracking testing (MAT) unit according to ASTM standard method D5154 entitled "Determining Activity and Selectivity of Fluid Catalytic Cracking (FCC) Catalysts by Microactivity Test." The FCC effluent, which included the effluents from catalytically cracking each of the greater boiling point fraction and lesser boiling point fraction, were collected and the properties and compositions analyzed according to known methods. Coke on the spent catalyst was determined for each FCC reaction using a Horiba carbon analyzer.

The composition of the FCC effluent obtained from the MAT unit is then used to model various configurations of the downstream unit operations of the system 100 for increasing conversion of the hydrocarbons from the feed into greater value products and intermediates, such as propylene and light aromatic compounds (e.g., benzene, xylenes). Referring to FIG. 1, the FCC effluent is first separated in the separation system 200 to produce the light products effluent 202, a mixed C4 effluent 203, the naphtha effluent 204, and the heavy effluent 205. The separation in the separation system is assumed to be 100% for each fraction. Various configurations of the downstream unit operations of the system 100 are evaluated. For each of the five configurations in Table 3, the downstream unit operations of the system 100 include the olefin separation system 300, the pyrolysis cracking system 400, the C4 processing system 500, the naphtha reforming system 600, the aromatics recovery complex 700, and the heavy oil processing system 800. Operating parameters and assumptions, such as but not limited to conversion, selectivities, energy consumption, separation efficiencies, and other operating assumptions, for each of the downstream unit operations are developed from historic operating data for each of the unit operations. For each configuration of the system 100, a mass balance and energy balance are conducted using a simulation software (e.g., Aspen 6) based on the FCC effluent composition obtained from the experimental data and the operating parameters and assumptions developed from the historic operating data.

For each configuration, a discounted cash flow model is then used to evaluate the economics of various configurations of the integrated HSFCC and refinery systems disclosed herein using the mass and energy balance. The discounted cash flow model incorporates estimates of capital expenditure (CAPEX) and utilities consumption. Overhead and other fixed costs were maintained constant for each of the configurations. The economic performance index is the net profitability index, which is net present value divided by the CAPEX. The top five configurations according to economic performance were selected and the results are provided in Table 3.

Configuration 1 provides the best economic performance, which may be attributed in part by the greater yields of propylene and aromatic compounds and reduced yield of ethylene, which is of lower economic value compared to propylene and aromatic compounds (e.g., benzene and xylenes). For configuration 1, the C4 processing system 500 includes the metathesis reactor 520 for conversion of butenes to C2-C3 olefins and the naphtha reforming system 600 includes the naphtha hydrotreater 610 and the naphtha reforming unit 620 downstream of the naphtha hydrotreater 610. The modeling information for Configurations 1-5 (i.e., ethylene production, propylene production, aromatics production, crude to chemicals conversion, and economic performance index) are provided in Table 3. Production for ethylene, propylene, and aromatics are provided in units of kilotons per annum (KTA).

**Table 3**

| **Configuration Number** | **1** | **2** | **3** | **4** | **5** |
|---|---|---|---|---|---|
| Ethylene Production (KTA) | 706 | 744 | 920 | 686 | 798 |
| Propylene Production (KTA) | 1899 | 1876 | 1564 | 1659 | 1465 |
| Aromatics Production (Benzene + Xylenes) (KTA) | 1538 | 1500 | 1475 | 1459 | 1436 |
| Crude to Chemicals Conversion (wt.%) | 76% | 77% | 74% | 73% | 71% |
| Economic Performance index | 2.04 | 2.03 | 1.98 | 1.99 | 1.98 |

A first aspect of the present disclosure is directed to an integrated process for upgrading a crude oil, where the process may comprise: processing a crude oil in a high severity fluidized catalytic cracking (HSFCC) system to produce an HSFCC effluent, wherein the HSFCC system may comprise a feed separator, a first FCC unit, and a second FCC unit in parallel with the first FCC unit; separating the HSFCC effluent in a separation system to produce, a light products effluent, a mixed C₄ effluent, a naphtha effluent, and a heavy effluent, where the light products effluent may comprise hydrocarbon constituents of the HSFCC effluent having a boiling point temperature less than or equal to -20 °C, the mixed C₄ effluent may comprise constituents of the HSFCC effluent having 4 carbon atoms, the naphtha effluent may comprise constituents of the HSFCC effluent having boiling point temperatures of from 25 °C to 220 °C, and the heavy effluent may comprise constituents of the HSFCC effluent having boiling point temperatures greater than 220 °C; passing the light products effluent to an olefin separation system that may separate the light products effluent to produce a C₂-C₃ olefin effluent and a light saturated hydrocarbon effluent, where the C₂-C₃ olefin effluent may comprise ethylene, propylene, or both; subjecting the light saturated hydrocarbon effluent to steam cracking in a pyrolysis cracking system to produce a cracking reaction effluent comprising ethylene, propylene, or both; passing at least a portion of the mixed C₄ effluent to a C₄ processing system that processes the mixed C₄ effluent to produce one or more of a saturated C₄ stream and a C₄ olefin stream; reforming the naphtha effluent in a naphtha reforming system to produce a reformate comprising a greater concentration of light aromatic compounds compared to the naphtha effluent, where the light aromatic compounds may comprise benzene, toluene, xylenes, ethylbenzene, or combinations thereof; recovering benzene, toluene, xylenes, ethylbenzene, or combinations thereof from the reformate; and passing at least a portion of the heavy effluent to heavy oil processing system that processes the heavy effluent to produce a light cycle oil (LCO) and a heavy cycle oil (HCO).

A second aspect of the present disclosure may include the first aspect, wherein at least 60 wt.% of the crude oil may be converted to light olefins and light aromatic compounds.

A third aspect of the present disclosure may include either one of the first or second aspect, further comprising separating the mixed C₄ effluent into the saturated C₄ stream and the C₄ olefin stream, wherein the saturated C₄ stream may comprise saturated C₄ hydrocarbons and the C₄ olefin stream comprises unsaturated C₄ hydrocarbons.

A fourth aspect of the present disclosure may include the third aspect, further comprising passing the saturated C₄ stream to the pyrolysis cracking system to produce ethylene, propylene, butenes, or a combination thereof.

A fifth aspect of the present disclosure may include either one of the third or fourth aspects, further comprising: passing the C₄ olefin stream to at least one metathesis reactor comprising at least one metathesis catalyst; and contacting the C₄ olefin stream with the at least one metathesis catalyst in the at least one metathesis reactor, where the contacting may produce ethylene, propylene, or combinations thereof.

A sixth aspect of the present disclosure may include either one of the third or fourth aspects, further comprising: passing the C₄ olefin stream and hydrogen to a hydrogenation reactor comprising a hydrogenation catalyst; contacting the C₄ olefin stream with hydrogen and the hydrogenation catalyst in the hydrogenation reactor, where the contacting may produce saturated C₄ hydrocarbons; and passing the saturated C₄ hydrocarbons to the pyrolysis cracking system.

A seventh aspect of the present disclosure may include any one of the first through sixth aspects, wherein the subjecting the light saturated hydrocarbon effluent to steam cracking in the pyrolysis cracking system may comprise: passing the light saturated hydrocarbon effluent and steam to a pyrolysis cracking reactor, wherein the pyrolysis cracking reactor may comprise a convection zone and a pyrolysis zone, wherein the light saturated hydrocarbon effluent may be preheated in the convection zone and may undergo steam cracking in the pyrolysis zone to produce a cracking reaction effluent, and wherein the cracking reaction effluent may comprise light olefins and intermediates; passing the cracking reaction effluent back to the olefin separation system; separating the light olefins from the intermediates in the olefin separation system; and passing the intermediates to the pyrolysis cracking reactor to undergo steam cracking to produce light olefins.

An eighth aspect of the present disclosure many include any one of the first through seventh aspects, wherein the reforming the naphtha effluent in the naphtha reforming system to produce the reformate may comprise: passing the naphtha effluent and hydrogen to a naphtha hydrotreater unit comprising at least one hydrotreating catalyst; contacting the naphtha effluent with hydrogen and the at least one hydrotreating catalyst, where the contacting may remove one or more impurities from the naphtha effluent to produce a hydrotreated naphtha; and passing the hydrotreated naphtha to a naphtha reforming unit that contacts the hydrotreated naphtha with at least one reforming catalyst, where the contacting may cause the hydrotreated naphtha to undergo one or more reforming reactions to produce the reformate.

A ninth aspect of the present disclosure may include the eighth aspect, further comprising: passing at least a portion of the reformate to an extraction unit to produce a raffinate comprising saturated hydrocarbons having greater than or equal to 5 carbon atoms; and passing the raffinate to the pyrolysis cracking system to produce light olefins.

A tenth aspect of the present disclosure may include the eighth aspect, further comprising: passing at least a portion of the reformate and to an aromatic recovery complex; and recovering a product comprising one or more of benzene, toluene, xylene, or combinations thereof from the at least a portion of the reformate.

An eleventh aspect of the present disclosure many include any one of the first through tenth aspects, further comprising: passing a portion of the LCO to an LCO hydrocracking unit comprising at least one hydrocracking catalyst; and contacting the LCO with hydrogen in the presence of the at least one hydrocracking catalyst, where the contacting may cause at least a portion of the LCO to undergo cracking to produce a naphtha stream, a diesel stream, a fuel oil stream, or a combination of these.

A twelfth aspect of the present disclosure may include the eleventh aspect, further comprising passing the naphtha stream to a naphtha reforming unit of the naphtha reforming system and contacting the naphtha stream with at least one reforming catalyst to produce the reformate.

A thirteenth aspect of the present disclosure may include any one of the first through twelfth aspects, further comprising passing at least a portion of the HCO to a heavy oil treatment unit that processes the heavy cycle oil to produce at least fuel oil.

A fourteenth aspect of the present disclosure may include any one of the first through thirteenth aspects, further comprising passing a portion of the LCO to the HSFCC system for heat balance.

A fifteenth aspect of the present disclosure may include any one of the first through fourteenth aspects, further comprising recycling a portion of the LCO to the HSFCC system.

A sixteenth aspect of the present disclosure may include any one of the first through fifteenth aspects, further comprising passing the HCO to the HSFCC system for heat balance.

A seventeenth aspect of the present disclosure may include any one of the first through sixteenth aspects, further comprising recycling a portion of the HCO to the HSFCC system.

An eighteenth aspect of the present disclosure may include any one of the first through seventeenth aspects, further comprising separating the crude oil into at least a greater boiling point fraction and a lesser point boiling fraction; passing the greater boiling point fraction to a first reactor of the HSFCC system that contacts the greater boiling point fraction in the presence of at least one cracking catalyst to produce a first cracking effluent and spent catalyst; and passing the lesser boiling point fraction to a second reactor of the HSFCC system that contacts the lesser boiling point fraction with the at least one cracking catalyst to produce a second cracking effluent and spent catalyst, wherein the HSFCC effluent may comprise the first and second cracking effluents.

A nineteenth aspect of the present disclosure may include the eighteenth aspect, further comprising: passing the spent catalyst to a regenerator, wherein the catalyst regenerator may be in fluid communication with a first reactor of the HSFCC system and the second reactor of the HSFCC system; regenerating the spent catalyst in the regenerator to form a regenerated catalyst; and passing the regenerated catalyst to the first and second reactors of the HSFCC system.

A twentieth aspect of the present disclosure many include any one of the first through nineteenth aspects, further comprising: separating the light products effluent to produce passing the light products effluent to produce a C₄ effluent; and passing the C₄ effluent to the C₄ processing system to produce one or more of a saturated C₄ stream and a C₄ olefin stream.

It is noted that any two quantitative values assigned to a property may constitute a range of that property, and all combinations of ranges formed from all stated quantitative values of a given property are contemplated in this disclosure.

It is noted that one or more of the following claims utilize the term "where" as a transitional phrase. For the purposes of defining the present technology, it is noted that this term is introduced in the claims as an open-ended transitional phrase that is used to introduce a recitation of a series of characteristics of the structure and should be interpreted in like manner as the more commonly used open-ended preamble term "comprising."

Having described the subject matter of the present disclosure in detail and by reference to specific aspects, it is noted that the various details of such aspects should not be taken to imply that these details are essential components of the aspects. Rather, the claims appended hereto should be taken as the sole representation of the breadth of the present disclosure and the corresponding scope of the various aspects described in this disclosure. Further, it will be apparent that modifications and variations are possible without departing from the scope of the appended claims.

## Claims

1. An integrated process for upgrading a crude oil, the process comprising:
processing a crude oil in a high severity fluidized catalytic cracking (HSFCC) system to produce an HSFCC effluent, wherein the HSFCC system comprises a feed separator, a first FCC unit, and a second FCC unit in parallel with the first FCC unit;
separating the HSFCC effluent in a separation system to produce, a light products effluent, a mixed C₄ effluent, a naphtha effluent, and a heavy effluent, where the light products effluent comprises hydrocarbon constituents of the HSFCC effluent having a boiling point temperature less than or equal to -20 °C, the mixed C₄ effluent comprises constituents of the HSFCC effluent having 4 carbon atoms, the naphtha effluent comprises constituents of the HSFCC effluent having boiling point temperatures of from 25 °C to 220 °C, and the heavy effluent comprises constituents of the HSFCC effluent having boiling point temperatures greater than 220 °C;
passing the light products effluent to an olefin separation system that separates the light products effluent to produce a C₂-C₃ olefin effluent and a light saturated hydrocarbon effluent, where the C₂-C₃ olefin effluent comprises ethylene, propylene, or both;
subjecting the light saturated hydrocarbon effluent to steam cracking in a pyrolysis cracking system to produce a cracking reaction effluent comprising ethylene, propylene, or both;
passing at least a portion of the mixed C₄ effluent to a C₄ processing system that processes the mixed C₄ effluent to produce one or more of a saturated C₄ stream and a C₄ olefin stream;
reforming the naphtha effluent in a naphtha reforming system to produce a reformate comprising a greater concentration of light aromatic compounds compared to the naphtha effluent, where the light aromatic compounds comprise benzene, toluene, xylenes, ethylbenzene, or combinations thereof;
recovering benzene, toluene, xylenes, ethylbenzene, or combinations thereof from the reformate; and
passing at least a portion of the heavy effluent to heavy oil processing system that processes the heavy effluent to produce a light cycle oil (LCO) and a heavy cycle oil (HCO).

2. The process of claim 1, wherein at least 60 wt.% of the crude oil is converted to light olefins and light aromatic compounds.

3. The process of either one of claims 1 or 2, further comprising separating the mixed C₄ effluent into the saturated C₄ stream and the C₄ olefin stream, wherein the saturated C₄ stream comprises saturated C₄ hydrocarbons and the C₄ olefin stream comprises unsaturated C₄ hydrocarbons, and passing the saturated C₄ stream to the pyrolysis cracking system to produce ethylene, propylene, butenes, or a combination thereof.

4. The process of claim 3, further comprising:
passing the C₄ olefin stream to at least one metathesis reactor comprising at least one metathesis catalyst; and
contacting the C₄ olefin stream with the at least one metathesis catalyst in the at least one metathesis reactor, where the contacting produces ethylene, propylene, or combinations thereof.

5. The process of claim 3, further comprising:
passing the C₄ olefin stream and hydrogen to a hydrogenation reactor comprising a hydrogenation catalyst;
contacting the C₄ olefin stream with hydrogen and the hydrogenation catalyst in the hydrogenation reactor, where the contacting produces saturated C₄ hydrocarbons; and
passing the saturated C₄ hydrocarbons to the pyrolysis cracking system.

6. The process of any one of claims 1-5, wherein the subjecting the light saturated hydrocarbon effluent to steam cracking in the pyrolysis cracking system comprises:
passing the light saturated hydrocarbon effluent and steam to a pyrolysis cracking reactor,
wherein the pyrolysis cracking reactor comprises a convection zone and a pyrolysis zone,
wherein the light saturated hydrocarbon effluent is preheated in the convection zone and undergoes steam cracking in the pyrolysis zone to produce a cracking reaction effluent, and
wherein the cracking reaction effluent comprises light olefins and intermediates,
passing the cracking reaction effluent back to the olefin separation system;
separating the light olefins from the intermediates in the olefin separation system; and
passing the intermediates to the pyrolysis cracking reactor to undergo steam cracking to produce light olefins.

7. The process of any one of claims 1-6, wherein the reforming the naphtha effluent in the naphtha reforming system to produce the reformate comprises:
passing the naphtha effluent and hydrogen to a naphtha hydrotreater unit comprising at least one hydrotreating catalyst;
contacting the naphtha effluent with hydrogen and the at least one hydrotreating catalyst, where the contacting removes one or more impurities from the naphtha effluent to produce a hydrotreated naphtha; and
passing the hydrotreated naphtha to a naphtha reforming unit that contacts the hydrotreated naphtha with at least one reforming catalyst, where the contacting causes the hydrotreated naphtha to undergo one or more reforming reactions to produce the reformate.

8. The process of claim 7, further comprising:
passing at least a portion of the reformate to an extraction unit to produce a raffinate comprising saturated hydrocarbons having greater than or equal to 5 carbon atoms; and
passing the raffinate to the pyrolysis cracking system to produce light olefins.

9. The process of claim 7, further comprising:
passing at least a portion of the reformate and to an aromatic recovery complex; and
recovering a product comprising one or more of benzene, toluene, xylene, or combinations thereof from the at least a portion of the reformate.

10. The process of any one of claims 1-9, further comprising:
passing a portion of the LCO to an LCO hydrocracking unit comprising at least one hydrocracking catalyst; and
contacting the LCO with hydrogen in the presence of the at least one hydrocracking catalyst, where the contacting causes at least a portion of the LCO to undergo cracking to produce a naphtha stream, a diesel stream, a fuel oil stream, or a combination of these.

11. The process of claim 10, further comprising passing the naphtha stream to a naphtha reforming unit of the naphtha reforming system and contacting the naphtha stream with at least one reforming catalyst to produce the reformate.

12. The process of any one of claims 1-11, further comprising passing at least a portion of the HCO to a heavy oil treatment unit that processes the heavy cycle oil to produce at least fuel oil, passing at least a portion of the HCO to the HSFCC system for heat balance, or both.

13. The process of any one of claims 1-12, further comprising passing a portion of the LCO to the HSFCC system for heat balance.

14. The process of any one of claims 1-13, further comprising:
separating the crude oil into at least a greater boiling point fraction and a lesser point boiling fraction;
passing the greater boiling point fraction to a first reactor of the HSFCC system that contacts the greater boiling point fraction in the presence of at least one cracking catalyst to produce a first cracking effluent and spent catalyst; and
passing the lesser boiling point fraction to a second reactor of the HSFCC system that contacts the lesser boiling point fraction with the at least one cracking catalyst to produce a second cracking effluent and spent catalyst, wherein the HSFCC effluent comprises the first and second cracking effluents.

15. The process of claim 14, further comprising:
passing the spent catalyst to a regenerator, wherein the catalyst regenerator is in fluid communication with a first reactor of the HSFCC system and the second reactor of the HSFCC system;
regenerating the spent catalyst in the regenerator to form a regenerated catalyst; and
passing the regenerated catalyst to the first and second reactors of the HSFCC system.

16. The process of any one of claims 1-15, further comprising:
separating the light products effluent to produce passing the light products effluent to produce a C₄ effluent; and
passing the C₄ effluent to the C₄ processing system to produce one or more of a saturated C₄ stream and a C₄ olefin stream.
